(19) Europäisches Patentamt
European Patent Office
Office européen des brevets

(11) Veröffentlichungsnummer: **0 174 654**
**A2**

# (12) EUROPÄISCHE PATENTANMELDUNG

(21) Anmeldenummer: **85111494.2**

(22) Anmeldetag: **11.09.85**

(51) Int.Cl.[4]: **C 07 D 211/90**
C 07 D 413/14, C 07 D 409/14
C 07 D 401/14, A 61 K 31/44

(30) Priorität: **14.09.84 CH 4388/84**

(43) Veröffentlichungstag der Anmeldung:
**19.03.86 Patentblatt 86/12**

(84) Benannte Vertragsstaaten:
**AT BE CH DE FR GB IT LI LU NL SE**

(71) Anmelder: **CIBA-GEIGY AG**
**Klybeckstrasse 141**
**CH-4002 Basel(CH)**

(72) Erfinder: **Zimmermann, Markus, Dr.**
**Steinbrecheweg 8**
**CH-4125 Riehen(CH)**

(74) Vertreter: **Zumstein, Fritz, Dr. et al,**
**Bräuhausstrasse 4**
**D-8000 München 2(DE)**

## (54) Carbonylverbindungen.

(57) Verbindungen der Formel

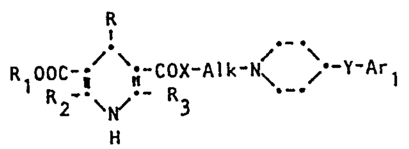

(I),

worin R einen carbocyclischen oder heterocyclischen Arylrest bedeutet, $R_1$ Niederalkyl darstellt, eine der Gruppen $R_2$ und $R_3$ für Niederalkyl und die andere für Niederalkyl, Cyan oder Amino steht, X Sauerstoff oder die Gruppe −NH− darstellt, und Alk Niederalkylen bedeutet, das die Gruppe X vom Ringstickstoffatom durch mindestens zwei Kohlenstoffatome trennt, Y für die Gruppen −CHOH− oder −(C=O)− steht und $Ar_1$ einen monocyclischen Aryl- oder Heteroarylrest darstellt, wobei, falls $R_1$, $R_2$ und $R_3$ jeweils Niederalkyl ist, X für Sauerstoff steht, Alk geradkettiges Alkylen mit 2 bis 4 Kohlenstoffatomen darstellt, Y die Gruppe −(C=O)− ist, und R die angegebene Bedeutung hat, $Ar_1$ verschieden von unsubstituiertem Phenyl ist, sowie Salze davon, zeigen cardiovaskuläre, insbesondere coronardilatatorische und antihypertensive Eigenschaften.

EP 0 174 654 A2

0174654

CIBA-GEIGY AG
Basel (Schweiz)

4-15072/+

# Carbonylverbindungen

Die Erfindung betrifft neue Carbonylverbindungen und deren Salze, Verfahren zu ihrer Herstellung, diese enthaltende pharmazeutische Präparate sowie deren Verwendung.

Die erfindungsgemässen Verbindungen entsprechen der Formel

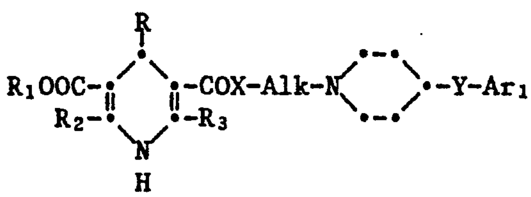

(I),

worin R einen carbocyclischen oder heterocyclischen Arylrest bedeutet, $R_1$ Niederalkyl darstellt, eine der Gruppen $R_2$ und $R_3$ für Niederalkyl und die andere für Niederalkyl, Cyan oder Amino steht, X Sauerstoff oder die Gruppe -NH- darstellt, und Alk Niederalkylen bedeutet, das die Gruppe X vom Ringstickstoffatom durch mindestens zwei Kohlenstoffatome trennt, Y für die Gruppen -CHOH- oder -(C=O)- steht, und $Ar_1$ einen monocyclischen Aryl- oder Heteroarylrest darstellt, wobei, falls $R_1$, $R_2$ und $R_3$ jeweils Niederalkyl ist, X für Sauerstoff steht, Alk geradkettiges Alkylen mit 2 bis 4 Kohlenstoffatomen darstellt, Y die Gruppe -(C=O)- ist, und R die angegebene Bedeutung hat, $Ar_1$ verschieden von unsubstituiertem Phenyl ist, sowie Salze von Verbindungen der Formel I.

Ein carbocyclischer oder heterocyclischer Arylrest R ist in erster Linie ein entsprechender monocyclischer Rest, kann jedoch auch ein bi- oder polycyclischer, carbocyclischer oder heterocyclischer Rest mit aromatischen Eigenschaften sein.

Carbocyclische Reste R dieser Art sind insbesondere gegebenenfalls substituiertes Phenyl, ferner Naphthyl.

Heterocyclische Arylreste R sind vorzugsweise entsprechende monocyclische Reste, können jedoch auch entsprechende bi- oder polycyclische Reste sein, wobei letztere aus mehreren heterocyclischen Ringen, oder aus einem oder mehreren Ringen mit einem oder mehreren ankondensierten carbocyclischen Ringen, insbesondere einem oder mehreren ankondensierten Benzoringen bestehen können. Die üblicherweise vorliegenden heterocyclischen Reste R, die vorzugsweise aus fünf oder sechs Ringgliedern bestehen, können bis zu vier, gleiche oder verschiedene Hetero-, insbesondere Stickstoff-, Sauerstoff- und/oder Schwefelatome, vorzugsweise ein, zwei, drei oder vier Stickstoffatome, ein Sauerstoff- oder Schwefelatom, oder ein oder zwei Stickstoffatome zusammen mit einem Sauerstoff- oder Schwefelatom als Ringglieder enthalten. Sie sind mit dem Kohlenstoffatom der 4-Stellung des 1,4-Dihydropyridinringes üblicherweise über ein Ringkohlenstoffatom gebunden.

Monocyclische fünfgliedrige Heteroarylreste R sind z.B. entsprechende monoaza-, diaza-, triaza-, tetraza-, monooxa-, monothia-, oxaza-, oxadiaza-, thiaza- oder thiadiazacyclische Reste, wie Pyrryl-, Pyrazolyl-, Imidazolyl-, Triazolyl-, Tetrazolyl-, Furyl-, Thienyl-, Isoxazolyl-, Oxazolyl-, Oxadiazolyl-, Isothiazolyl-, Thiazolyl- oder Thiadiazolylreste, während monocyclische, sechsgliedrige Heteroarylreste R z.B. entsprechende monoaza-, diaza- oder triazacyclische Reste, wie Pyridyl-, Pyridazinyl-, Pyrimidinyl-, Pyrazinyl- oder Triazinylreste sind. Bicyclische Heteroarylreste sind insbesondere monocyclische Heteroarylreste mit ankondensiertem Benzoring; der Heteroring kann fünf- oder sechsgliedrig sein, wobei

ein fünfgliedriger Heteroarylrest z.B. einen monoaza-, diaza-, diaza-monooxa-, monooxa-, monothia-, oxaza- oder thiazacyclischen Rest, und ein sechsgliedriger Heteroarylrest z.B. einen monoaza- oder einen diazacyclischen Heteroarylrest darstellt. Solche bicyclischen Reste, die über ein Ringkohlenstoffatom des hetero- oder carbocyclischen Restes gebunden sein können, sind z.B. Indolyl-, Isoindolyl-, Benzimidazolyl-, Benzoxadiazolyl-, Benzofuranyl-, Benzofurazanyl-, Benzothienyl-, Benzthiazolyl-, Benzthiadiazolyl-, Chinolinyl- oder Isochinolinylreste, wobei stickstoffhaltige mono- oder bicyclische Heteroarylreste der genannten Art, insbesondere unsubstituierte oder substituierte Pyridylreste, auch als N-Oxide vorliegen können, z.B. Pyridyl-N-oxid.

Ein monocyclischer, carbocyclischer Arylrest $Ar_1$ stellt in erster Linie einen unsubstituierten oder ein- oder mehrfach substituierten Phenylrest dar, während ein monocyclischer Heteroarlyrest $Ar_1$ beispielsweise einen monocyclischen Monooxa-, Monoaza- oder Monothiaarylrest, etwa solche, wie für R definiert, in erster Linie unsubstituiertes oder ein- oder mehrfach gleich oder verschieden substituiertes Furyl, Pyrryl, Pyridyl bzw. Thienyl bedeutet.

Die carbocyclischen und heterocyclischen Arylreste R sowie $Ar_1$ können unsubstituiert oder substituiert sein, wobei in erster Linie Ringkohlenstoffatome, aber auch Ringstickstoffatome substituiert, ein- bis dreifach vorhanden und gleich oder verschieden, vorzugsweise jedoch einfach vorhanden sein können. Substituenten von Ringkohlenstoffatomen sind u.a. gegebenenfalls substituierte Kohlenwasserstoffreste, wie entsprechende aliphatische, cycloaliphatische, aromatische oder araliphatische Kohlenwasserstoffreste, z.B. Niederalkyl, Niederalkenyl, Niederalkinyl, Niederalkylen, Cycloalkyl, Cycloalkylniederalkyl, Phenyl oder Phenylniederalkyl. Substituenten von solchen Kohlenwasserstoffresten, insbesondere von Niederalkyl, Phenyl oder Phenylniederalkyl, sind z.B. gegebenenfalls verätherte oder veresterte Hydroxygruppen, wie Hydroxy, gegebenenfalls, z.B. durch gegebenenfalls veräthertes oder verestertes Hydroxy substituiertes Niederalkoxy, z.B. Niederalkoxy,

Niederalkoxyniederalkoxy, oder Halogenniederalkoxy, gegebenenfalls, z.B. durch gegebenenfalls veräthertes oder verestertes Hydroxy, substituiertes Niederalkenyloxy, z.B. Niederalkenyloxy oder Halogenniederalkenyloxy, Niederalkinyloxy, Niederalkylendioxy, Niederalkanoyloxy oder Halogen, und/oder gegebenenfalls funktionell abgewandeltes Carboxy, wie Carboxy, verestertes Carboxy, z.B. Niederalkoxycarbonyl, amidiertes Carboxy, wie Carbamoyl, N-Niederalkyl-carbamoyl oder N,N-Diniederalkyl-carbamoyl, oder Cyan. Cyclische Substituenten, insbesondere Phenyl, können zudem auch Niederalkyl, das gegebenenfalls, z.B. wie angegeben, substituiert sein kann, als Substituenten enthalten. Weitere Substituenten von Arylresten R sowie $Ar_1$ sind z.B. gegebenenfalls verätherte oder veresterte Hydroxygruppen, wie Hydroxy, gegebenenfalls, z.B. durch gegebenenfalls veräthertes oder verestertes Hydroxy substituiertes Niederalkoxy, z.B. Niederalkoxy, Niederalkoxyniederalkoxy oder Halogenniederalkoxy, gegebenenfalls, z.B. durch gegebenenfalls veräthertes oder verestertes Hydroxy substituiertes Niederalkenyloxy, z.B. Niederalkenyloxy oder Halogenniederalkenyloxy, Niederalkinyloxy, Niederalkylendioxy, Niederalkanoyloxy oder Halogen, Nitro, gegebenenfalls substituiertes Amino, wie Amino, N-Niederalkylamino, N,N-Diniederalkylamino, N-Niederalkyl-N-phenylniederalkylamino, Niederalkylenamino, Oxaniederalkylenamino, Thianiederalkylenamino oder Azaniederalkylenamino, wobei der Aza-Stickstoff unsubstituiert oder, z.B. durch gegebenenfalls, z.B. wie oben beschrieben, substituiertes Niederalkyl, Phenyl oder Phenylniederalkyl substituiert sein kann, oder Acylamino, z.B. Niederalkanoylamino, Azido, Acyl, wie Niederalkanoyl oder gegebenenfalls funktionell abgewandeltes Carboxy, wie Carboxy, verestertes Carboxy, z.B. Niederalkoxycarbonyl, oder amidiertes Carboxy, wie Carbamoyl, N-Niederalkyl-carbamoyl oder N,N-Diniederalkyl-carbamoyl, ferner Cyan, gegebenenfalls funktionell abgewandeltes Sulfo, wie Sulfo, Aminosulfonyl, N-Niederalkylaminosulfonyl oder N,N-Diniederalkylaminosulfonyl und/oder veräthertes Mercapto, das gegebenenfalls oxidiert sein kann, wie Niederalkylthio, Niederalkylsulfinyl oder

Niederalkylsulfonyl. Substituenten von Ringstickstoffatomen sind in erster Linie die obengenannten, gegebenenfalls substituierten Kohlenwasserstoffreste, wie Niederalkyl, ferner Hydroxy oder Oxido.

Heterocyclische Arylreste R bzw. $Ar_1$ können, z.B. je nach Art der Substitution, in verschiedenartigen tautomeren Formen vorliegen.

Der Alkylenrest Alk trennt die Gruppe X vom Ringstickstoffatom vorzugsweise durch 2 bis 8 Kohlenstoffatome und ist z.B. Aethylen, 1,2- oder 1,3-Propylen, 1,4-Butylen, 1,5-Pentylen, oder 1,6-Hexylen.

Die vor-, sowie nachstehend verwendeten Definitionen haben, sofern nicht besonders definiert, die folgenden Bedeutungen:

Der Ausdruck "nieder" bedeutet, dass entsprechend definierte Gruppen oder Verbindungen, sofern nicht anders definiert, bis und mit 7, vorzugsweise bis und mit 4, Kohlenstoffatome enthalten.

Substituierte Reste können einen oder mehrere, gleiche oder verschiedene Substituenten enthalten; diese können irgendeine geeignete Stellung einnehmen.

Naphthyl kann 1- oder 2-Naphthyl sein.

Pyrryl ist z.B. 2- oder 3-Pyrryl, Pyrazolyl z.B. 3- oder 4-Pyrazolyl, Imidazolyl z.B. 2- oder 4-Imidazolyl, Triazolyl z.B. 1,3,5-1H-Triazol-2-yl oder 1,3,4-Triazol-2-yl, und Tetrazolyl z.B. 1,2,3,4-1H-Tetrazol-5-yl, während Furyl 2- oder 3-Furyl und Thienyl 2- oder 3-Thienyl bedeuten. Isoxazolyl ist z.B. 3-Isoxazolyl, Oxazolyl z.B. 2- oder 4- Oxazolyl, Oxadiazolyl z.B. 1,3,4-Oxadiazol-2-yl, Isothiazolyl z.B. 3-Isothiazolyl, Thiazolyl 2- oder 4-Thiazolyl, und Thiadiazolyl z.B. 1,3,4-Thiadiazolyl-2-yl.

Pyridyl ist 2-, 3- oder 4-Pyridyl, Pyridazinyl z.B. 3-Pyridazinyl, Pyrimidinyl 2-, 4- pder 5-Pyrimidinyl, Pyrazinyl, 2-Pyrazinyl und Triazinyl z.B. 1,3,5-Triazin-2-yl.

Indolyl ist z.B. 2-, 3- oder 5-Indolyl, Isoindolyl z.B. 1-Isoindolyl, Benzimidazolyl z.B. 2- oder 5-Benzimidazolyl, Benzofuranyl z.B. 2- oder 3-Benzofuranyl, Benzofurazanyl (auch 2,1,3-Benzoxadiazolyl) z.B. 4-Benzofurazanyl, Benzothienyl z.B. 3-Benzothienyl, Benzthiazolyl z.B. 2-Benzthiazolyl, 2,1,3-Benzthiadiazolyl z.B. 2,1,3-Benzthiadiazol-4-yl, Chinolinyl z.B. 2- oder 4-Chinolinyl, und Isochinolinyl z.B. 1-Isochinolinyl.

Niederalkyl ist z.B. Methyl, Aethyl, n-Propyl, Isopropyl, n-Butyl, Isobutyl oder tert.-Butyl, ferner n-Pentyl, n-Hexyl oder n-Heptyl, während Niederalkenyl z.B. Allyl oder Methallyl, und Niederalkinyl z.B. Propargyl bedeutet.

Cycloalkyl weist vorzugsweise 5 bis 7 Ringkohlenstoffatome auf und ist z.B. Cyclopentyl oder Cyclohexyl, während Cycloalkylniederalkyl z.B. Cyclopropylmethyl, Cyclopentylmethyl oder Cyclohexylmethyl sein kann.

Phenylniederalkyl ist z.B. Benzyl oder 1- oder 2-Phenyläthyl.

Niederalkoxy steht in erster Linie für Methoxy, Aethoxy, n-Propyloxy, Isopropyloxy, n-Butyloxy, Isobutyloxy oder tert.-Butyloxy.

In einem Niederalkoxyniederalkoxyrest ist die terminale Niederalkoxygruppe vorzugsweise durch mehr als ein Kohlenstoffatom vom Verknüpfungssauerstoffatom getrennt; solche Reste sind z.B. 2-Methoxyäthoxy oder 2-Aethoxy-äthoxy.

In einem Halogen-niederalkoxyrest können ein oder mehrere Halogenatome, die vorzugsweise eine Atomnummer bis 35 aufweisen und besonders für Fluor und/oder Chlor stehen, vorhanden sein; solche Reste sind z.B. Difluormethoxy oder 1,1,2-Trifluor-2-chlor-äthoxy.

Niederalkenyloxy ist z.B. Allyloxy oder Methallyloxy, und Halogenniederalkenyloxy, das ein oder mehrere Halogenatome enthalten kann, wobei letztere vorzugsweise eine Atomnummer bis 35 aufweisen und besonders für Fluor und/oder Chlor stehen, ist z.B. 1,2-Dichlorvinyloxy.

Niederalkinyloxy ist z.B. Propargyloxy, während Niederalkylendioxy z.B. Methylendioxy oder Aethylendioxy bedeutet.

Niederalkylen ist z.B. 1,2-Aethylen oder 1,3-Propylen, während Niederalkylendioxy z.B. Methylendioxy oder 1,2-Aethylendioxy ist.

Niederalkanoyloxy ist z.B. Acetyloxy, Propionyloxy oder Pivaloyloxy.

Halogen hat vorzugsweise eine Atomnummer bis und mit 35 und ist insbesondere Fluor oder Chlor, ferner Brom, kann jedoch auch Jod sein.

Halogensubstituiertes Niederalkyl ist z.B. Trifluormethyl, 1,1,2-Trifluor-2-chlor-äthyl oder Chlormethyl.

Niederalkoxycarbonyl ist z.B. Methoxycarbonyl, Aethoxycarbonyl, n-Propyloxycarbonyl, Isopropyloxycarbonyl, n-Butyloxycarbonyl, Isobutyloxycarbonyl oder tert.-Butyloxycarbonyl.

N-Niederalkyl-carbamoyl ist z.B. N-Methyl-carbamoyl oder N-Aethylcarbamoyl, während N,N-Diniederalkyl-carbamoyl z.B. N,N-Dimethylcarbamoyl oder N,N-Diäthyl-carbamoyl ist.

N-Niederalkylamino ist z.B. N-Methylamino, N-Aethylamino, N-n-Propylamino oder N-Isopropylamino.

N,N-Diniederalkylamino ist z.B. N,N-Dimethylamino, N-Aethyl-N-methylamino oder N,N-Diäthylamino, während N-Niederalkyl-N-phenylniederalkylamino z.B. N-Benzyl-N-methylamino oder N-methyl-N-(2-phenyläthyl)-amino darstellt.

Niederalkylenamino enthält vorzugsweise 4 bis 6 Ringkohlenstoffatome und ist z.B. Pyrrolidino oder Piperidino, während Oxaniederalkylenamino z.B. 4-Morpholino, Thianiederalkylenamino z.B. 4-Thiomorpholino, und gegebenenfalls azasubstituiertes Azaniederalkylenamino z.B. Piperazino, 4-Methylpiperazino, 4-Phenyl-piperazino, 4-Benzyl-piperazino oder 4-(2-Phenyläthyl)-piperazino darstellen kann.

Niederalkanoylamino ist z.B. Acetylamino oder Propionylamino.

Niederalkanoyl ist z.B. Formyl, Acetyl, Propionyl oder Pivaloyl.

Niederalkylthio ist z.B. Methylthio, Aethylthio, n-Propylthio oder Isopropylthio, während Niederalkylsulfinyl z.B. Methylsulfinyl, und Niederalkylsulfonyl z.B. Methylsulfonyl oder Aethylsulfonyl bedeuten.

N-Niederalkylaminosulfonyl ist z.B. N-Methylaminosulfonyl, während N,N-Diniederalkylaminosulfonyl z.B. N,N-Dimethylaminosulfonyl ist.

In einem substituierten Niederalkoxycarbonyl ist der Substituent üblicherweise durch mindestens 2, vorzugsweise durch 2 oder 3 Kohlenstoffatome vom Sauerstoff getrennt. Solche Reste sind z.B. Hydroxyniederalkoxycarbonyl, wie 2-Hydroxyäthoxycarbonyl oder 2,3-Dihydroxypropyloxycarbonyl, Niederalkoxy-niederalkoxycarbonyl, z.B. 2-Methoxyäthoxycarbonyl, Diniederalkylamio-niederalkoxycarbonyl, z.B. 2-Dimethylaminoäthoxycarbonyl, 2-Diäthylaminoäthoxycarbonyl oder 3-Dimethylaminopropyloxycarbonyl, Niederalkylenaminoniederalkoxycarbonyl, z.B. 2-Pyrrolidinoäthoxycarbonyl oder 2-Piperidinoäthoxycarbonyl, Morpholino-niederalkoxycarbonyl, z.B. 2-(4-Morpholino)-äthoxycarbonyl, oder (4-Niederalkyl-piperazino)-n-niederalkoxycarbonyl, z.B. 2-(4-Methylpiperazino)-äthoxycarbonyl.

Phenylniederalkoxycarbonyl ist z.B. Benzyloxycarbonyl oder 2-Phenyläthoxy-carbonyl.

N,N-Niederalkylen-carbamoyl ist z.B. Pyrrolidinocarbonyl oder Piperidinocarbonyl, wobei entsprechende Reste, in welchen der Niederalkylenteil durch Sauerstoff, Schwefel oder unsubstituierten oder substituierten Stickstoff unterbrochen ist, z.B. 4-Morpholinocarbonyl, 4-Thiomorpholinocarbonyl, 1-Piperazinocarbonyl oder 4-Methyl-1-piperazinocarbonyl bedeutet.

Verbindungen der Formel I können in Form von Salzen, insbesondere von Säureadditionssalzen, in erster Linie entsprechenden pharmazeutisch verwendbaren, nicht-toxischen Säureadditionssalzen, vorliegen. Solche Salze sind z.B. diejenigen mit Halogenwasserstoffsäuren, z.B. Chlorwasserstoffsäure oder Bromwasserstoffsäure, ferner Salpetersäure, Schwefelsäure oder Phosphorsäure, oder organischen Säuren, wie Carbonsäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure oder Isonicotinsäure, ferner Aminosäuren, oder organischen Sulfonsäuren, wie gegebenenfalls Hydroxy enthaltende Niederalkansulfonsäuren, z.B. Methansulfonsäure, Aethansulfonsäure, 2-Hydroxyäthansulfonsäure oder Aethan-1,2-disulfonsäure, oder Arylsulfonsäure, z.B. Benzolsulfonsäure, 4-Methyl-benzolsulfonsäure oder Naphthalin-2-sulfonsäure, oder mit anderen sauren organischen Substanzen, wie Ascorbinsäure.

Die Verbindungen der Formel I und deren Salze besitzen wertvolle pharmakologische Eigenschaften, vor allem im cardiovasculären Bereich. Sie wirken als Calcium-Antagonisten und besitzen α-Rezeptoren blockierende sowie Serotonin-antagonistische Eigenschaften, wie sich z.B. in in-vitro-Versuchen am isoliert perfundierten Mesenterialbett der Ratte [Mc Gregor D.D.: J.Physiol. 177, 21-30, (1965)] als Hemmung der Calcium-,Noradrenalin- und Serotonininduzierten Vasokonstriktion, beispielsweise unter Verwendung von 2,6-Dimethyl-4-(3-nitrophenyl)-1,4-dihydropyridin-3,5-dicarbonsäure-methylester-2-[4-(p-fluorbenzoyl)-piperidino]-äthylester-hydrochlorid oder von 2,6-Dimethyl-4-(3-nitrophenyl)-1,4-dihydropyridin-3-carbonsäuremethylester-5-N-[2-[4(p-fluorbenzoyl)-piperidino]-äthyl]-carboxamid-hydrochlorid in einem Konzentrationsbereich von etwa 5 x $10^{-10}$ Mol/Liter bis etwa $10^{-8}$ Mol/Liter zeigen lässt.

Ferner lässt sich die Bindung der neuen Verbindungen oder deren Salze an Dihydropyridin-Rezeptoren mittels Verdrängung der Bindung von $^3$H-Nitrendipin an Membranen von Meerschweichenherzen in in-vitro-Versuchen [Erne P. et al,: Biochem., Biophys. Res. Commm., 118, 842-847 (1984)] z.B. unter Verwendung von 2,6-Dimethyl-4-(3-nitrophenyl)-1,4-dihydropyridin-3,5-dicarbonsäure-methylester-2-[4-(p-fluorbenzoyl)-piperidino]-äthylester-hydrochlorid oder von 2,6-Dimethyl-4-(3-nitrophenyl)-1,4-dihydropyridin-3-carbonsäure-methyl ester-5-N-[2-[4-(p-fluorbenzoyl)-piperidino]-äthyl]-carboxamid-hydrochlorid in einem Konzentrationsbereich von etwa $10^{-10}$ Mol/Liter bis etwa $10^{-7}$ Mol/Liter nachweisen. Die neuen Verbindungen oder deren Salze wirken ausserdem antihypertensiv, wie sich z.B. durch Senkung des Blutdrucks an der renal hypertensiven Ratte in einem Dosisbereich von etwa 1-100 mg/kg p.o., zeigen lässt.

So kann z.B. 2 Stunden nach p.o.-Applikation von 60 mg/kg 2,6-Dimethyl-4-(3-nitrophenyl)-1,4-dihydropyridin-3-carbonsäuremethylester-5-N-[2-[4-(p-fluorbenzoyl)-piperidino]-äthyl]-carboxamid-hydrochlorid an der renal hypertensiven Ratte eine Blutdrucksenkung von ca. -50 mm Hg beobachtet werden.

Die Verbindungen der Formel I und Salze von solchen Verbindungen können daher z.B. als Coronardilatatoren und Antihypertensiva zur Behandlung von cardiovasculären Krankheitszuständen, wie Angina pectoris und ihre Folgen, Gefässspasmen, zentrale und periphere Durchblutungsstörungen, hohem Blutdruck, Arrhythmien und Herzinsuffizienz, ferner zur Hemmung der Plättchenaggregation Verwendung finden. Die neuen Verbindungen sind aber auch wertvolle Zwischenprodukte zur Herstellung anderer, insbesondere pharmazeutisch wirksamer Verbindungen.

Die Erfindung betrifft insbesondere neue Verbindungen der Formel I, in welchen R für einen mono- oder bicyclischen, carbocyclischen Arylrest oder für einen fünf- oder sechsgliedrigen, ein bis und mit vier Ringstickstoffatome, ein Ringsauerstoff- oder Ringschwefelatom, oder ein oder zwei Ringstickstoffatome zusammen mit einem Ringsauerstoff- oder einem Ringschwefelatom als Ringglieder enthaltenden, monocyclischen Heteroarylrest steht, der über ein Ringkohlenstoffatom mit dem Kohlenstoffatom der 4-Stellung des 1,4-Dihydropyridinrings verbunden ist, und der gegebenenfalls einen ankondensierten Benzoring enthält, und insbesondere Phenyl, Naphthyl, Pyrryl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl, Triazinyl, Indolyl, Isoindolyl, Benzimidazolyl, Benzoxadiazolyl, Benzofuranyl, Benzofurazanyl, Benzothienyl, Benzthiazolyl, 2,1,3-Benzthiadiazolyl, Chinolinyl oder Isochinolinyl bedeutet, wobei in diesen Resten Ringkohlenstoffatome gegebenenfalls durch Niederalkyl, Niederalkenyl, Niederalkinyl, Niederalkylen, Cycloalkyl, Phenyl und/oder Phenylniederalkyl, wobei Niederalkyl, Phenyl oder Phenylniederalkyl gegebenenfalls Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl und/oder Cyan, und cyclische Reste auch Niederalkyl, das seinerseits wie angegeben substituiert sein kann, als Substituenten enthalten können, und/oder durch Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Nitro, Amino, N-Niederalkyl-amino, N,N-Diniederalkylamino, N-Niederalkyl-N-phenylniederalkyl-amino, Niederalkylenamino, Oxaniederalkylenamino, Thianiederalkylenamino und/oder Azaniederalkylenamino, worin das Azastickstoffatom durch Niederalkyl, Phenyl oder Phenylniederalkyl substituiert sein kann, wobei diese Reste Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl und/oder Cyan, die cyclischen Reste auch Niederalkyl als Substituenten enthalten können, und/oder durch Niederalkanoylamino, Azido, Niederalkanoyl, Carboxy, Niederalkoxycarbonyl, Carbamoyl, N-Niederalkyl-carbamoyl, N,N-Diniederalkyl-carbamoyl, Cyan, Sulfo, Aminosulfonyl, N-Niederalkylaminosulfonyl, N,N-Diniederalkylaminosulfonyl, Niederalkylthio, Niederalkylsulfinyl und/oder Niederalkylsulfonyl, und/oder Ringstickstoffatome gegebenenfalls durch Niederalkyl, das als Substituenten gegebenenfalls Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkylcarbamoyl, N,N-Diniederalkyl-carbamoyl oder Cyan enthalten kann, oder durch Hydroxy oder Oxido substituiert sein können, $R_1$ Niederalkyl ist, einer der Reste $R_2$ und $R_3$ für Niederalkyl und der andere für Niederalkyl, Cyan oder Amino steht, X Sauerstoff oder die Gruppe -NH- bedeutet, und Alk Niederalkylen darstellt, das die Gruppe X vom Ringstickstoffatom durch 2 bis 8 Kohlenstoffatome trennt, Y für die Gruppen der Formel -CHOH- oder insbesondere -(C=O)- steht, und $Ar_1$ einen monocyclischen carbocyclischen Aryl- oder Heteroarylrest darstellt, und insbesondere Phenyl, Naphthyl, Pyrryl, Furyl, Thienyl oder Pyridyl bedeutet, wobei in diesen Resten Ringkohlenstoffatome gegebenenfalls durch Niederalkyl, Niederalkenyl, Niederalkinyl, Niederalkylen, Cycloalkyl, Phenyl und/oder Phenylniederalkyl substituiert sind, wobei Niederalkyl, Phenyl oder Phenylniederalkyl als Substituenten Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkylcarbamoyl und/oder Cyan, und cyclische Reste auch Niederalkyl, das seinerseits wie angegeben substituiert sein kann, enthalten können, und/oder durch Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen,

Nitro, Amino, N-Niederalkyl-amino, N,N-Diniederalkylamino, N-Niederalkyl-N-phenylniederalkyl-amino, Niederalkylenamino, Oxoniederalkylenmino, Thianiederalkylenamino und/oder Azaniederalkylenamino, worin das Azastickstoffatom durch Niederalkyl, Phenyl oder Phenylniederalkyl substituiert sein kann, wobei diese Reste Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkylcarbamoyl und/oder Cyan, die cyclischen Reste auch Niederalkyl als Substituenten enthalten können, und/oder durch Niederalkanoylamino, Azido, Niederalkanoyl, Carboxy, Niederalkoxycarbonyl, Carbamoyl, N-Niederalkyl-carbamoyl, N,N-Diniederalkyl-carbamoyl, Cyan, Sulfo, Aminosulfonyl, N-Niederalkylaminosulfonyl, N,N-Diniederalkylaminosulfonyl, Niederalkylthio, Niederalkylsulfinyl und/oder Niederalkylsulfonyl, und/oder Ringstickstoffatome gegebenenfalls durch Niederalkyl, das als Substituenten gegebenenfalls Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkylcarbamoyl, N,N-Diniederalkyl-carbamoyl oder Cyan als Substituenten enthalten kann, oder durch Hydroxy oder Oxido substituiert sein können, wobei falls $R_1$, $R_2$ und $R_3$ jeweils Niederalkyl ist, X für Sauerstoff steht, Alk geradkettiges Alkylen mit 2 bis 4 Kohlenstoffatomen darstellt, Y die Gruppe -(C=O)- ist, und R die angegebene Bedeutung hat, $Ar_1$ verschieden von unsubstituiertem Phenyl ist, sowie Salze insbesondere pharmazeutisch verwendbare, nichttoxische Säureadditionssalze solcher Verbindungen.

Die Erfindung betrifft in erster Linie neue Verbindungen der Formel I, worin R und $Ar_1$ jeweils für Phenyl oder Naphthyl stehen, das gegebenenfalls durch Niederalkyl, Phenyl und/oder Phenylniederalkyl, wobei solche Reste ihrerseits Hydroxy, Niederalkoxy, Halogenniederalkoxy, Niederalkylendioxy, Halogen, Carboxy, Niederalkoxycarbonyl und/oder Cyan, die cyclischen Reste auch Niederalkyl als Substituenten enthalten können, und/oder durch Hydroxy, Niederalkoxy, Halogenniederalkoxy, Niederalkenyloxy, Halogenniederalkenyloxy, Niederalkylendioxy, Halogen, Nitro, Amino, N-Niederalkylamino, N,N-Diniederalkylamino, Niederalkanoylamino, Carboxy, Niederalkoxycarbonyl, Carbamoyl, Cyan, Sulfo, Aminosulfonyl, N-Niederalkylaminosulfonyl, N,N-Diniederalkylaminosulfonyl, Niederalkylthio, Niederalkylsulfinyl und/oder Niederalkylsulfonyl substituiert ist, R zudem für über ein Ringkohlenstoffatom gebundenes Pyrryl, Furyl, Thienyl, Pyridyl, 1-Oxido-pyridyl, Imidazolyl, Benzofurazanyl oder Benzoxadiazolyl und $Ar_1$ zudem für über ein Ringkohlenstoffatom gebundenes Pyrryl, Furyl, Thienyl oder Pyridyl stehen kann, wobei solche Reste R und/oder $Ar_1$ gegebenenfalls, wie für einen Phenyl- oder Naphthylrest R bzw. $Ar_1$ angegeben, substituiert sind, und insbesondere Niederalkyl, Niederalkoxy, Halogen und/oder gegebenenfalls durch Niederalkyl, Niederalkoxy, Halogen und/oder Nitro substituiertes Phenyl als Substituenten enthalten können, $R_1$ Niederalkyl ist, $R_2$ und $R_3$ jeweils Niederalkyl bedeuten, oder eine der Gruppen $R_2$ und $R_3$ Niederalkyl ist und die andere Niederalkyl oder Cyan darstellt, X vorzugsweise Sauerstoff oder die Gruppe -NH- bedeutet, und Alk für die Gruppe $-(CH_2)_n-$ steht, worin n Werte von 2 bis 6 darstellt, und Y für die Gruppen der Formeln -CHOH- oder in erster Linie -(C=O)- steht, wobei, falls $R_1$, $R_2$ und $R_3$ jeweils Niederalkyl ist, X für Sauerstoff steht, n Werte von 2 bis 4 darstellt, Y die Gruppe -(C=O)- ist, und R die angegebene Bedeutung hat, $Ar_1$ verschieden von unsubstituiertem Phenyl ist, sowie Salze, insbesondere pharmazeutisch verwendbare, nichttoxische Säureadditionssalze solcher Verbindungen.

Die Erfindung betrifft ganz besonders neue Verbindungen der Formel I, worin R und $Ar_1$ jeweils für Phenyl steht, das gegebenenfalls durch Niederalkyl, z.B. Methyl, Hydroxy, Niederalkoxy, z.B. Methoxy oder Aethoxy, Halogenniederalkoxy, z.B. Difluormethoxy oder 2-Chlor-1,1,2-trifluoräthoxy, Halogenniederalkenyloxy, z.B. 1,2-Dichlor-vinyloxy, Niederalkylendioxy, z.B. Methylendioxy, Halogen, z.B. Fluor, Chlor oder Brom, Trifluormethyl, Nitro, Niederalkanoylamino, z.B. Acetylamino, gegebenenfalls durch gegebenenfalls verestertes oder veräthertes Hydroxy, wie Halogen,

z.B. Fluor oder Chlor, oder Niederalkoxy, z.B. Methoxy, substituiertes Phenyl oder Phenylniederalkyl, und/oder Cyan substituiert ist, wobei ein Phenylrest R bzw. $Ar_1$ einen oder mehrere, gleiche oder verschiedene Substituenten aufweisen kann, R zudem Pyridyl, z.B. 2-, 3- oder 4-Pyridyl, Furyl, z.B. 2-Furyl, 1-Oxido-pyridyl, z.B. 1-Oxido-3-pyridyl, Thienyl, z.B. 2-Thienyl, Benzofurazanyl, z.B. 4-Benzofurazanyl, und $Ar_1$ zudem Pyridyl, z.B. 2-, 3- oder 4-Pyridyl, Furyl, z.B. 2-Furyl, oder Thienyl, z.B. 2-Thienyl bedeuten kann, wobei solche Reste R bzw. $Ar_1$ durch Niederalkyl, z.B. Methyl, oder Halogen, z.B. Fluor oder Chlor, substituiert sein können, $R_1$, $R_2$ und $R_3$ jeweils Niederalkyl bedeuten, X für insbesondere Sauerstoff, ferner für die Gruppe -NH- steht, Alk die Gruppe $-(CH_2)_n-$ darstellt, worin n Werte von 2 bis 4 bedeutet, und Y für die Gruppen der Formeln -CHOH- oder insbesondere -(C=O)- steht, wobei falls $R_1$, $R_2$ und $R_3$ jeweils Niederalkyl ist, X für Sauerstoff steht, n Werte von 2 bis 4 darstellt, Y die Gruppe -(C=O)- ist, und R die angegebene Bedeutung hat, $Ar_1$ verschieden von unsubstituiertem Phenyl ist, sowie Salze, insbesondere pharmazeutisch verwendbare nicht-toxische Säureadditonssalze solcher Verbindungen.

Die Erfindung betrifft insbesondere neue Verbindungen der Formel I, worin R für unsubstituiertes oder vorzugsweise durch Niederalkyl, z.B. Methyl, Niederalkoxy, z.B. Methoxy, Halogenniederalkoxy oder Halogenniederalkenyloxy, worin Halogen eine Atomnummer bis und mit 35 hat und insbesondere Fluor oder Chlor ist, z.B. Difluormethoxy, Halogen mit einer Atomnummer bis und mit 35, Trifluormethyl, Nitro und/oder Cyan mono- oder di-substituiertes Phenyl steht, wobei Substituenten üblicherweise die 2- und/oder 3-Stellung einnehmen, $R_1$, $R_2$ und $R_3$ jeweils Niederalkyl bedeuten, X insbesondere für Sauerstoff, ferner für die Gruppe -NH- steht, Alk die Gruppe $-(CH_2)_n-$ darstellt, worin n Werte von 2 bis 4 bedeutet, Y für die Gruppen der Formeln -CHOH- oder insbesondere -(C=O)- steht, und $Ar_1$ gegebenenfalls durch Halogen mit einer Atomnummer bis und mit 35 substituiertes Phenyl oder Pyridyl, z.B. 2-Pyridyl, 3-Pyridyl oder 4-Pyridyl, bedeutet, wobei, falls $R_1$, $R_2$ und $R_3$ jeweils Niederalkyl ist, X für Sauerstoff steht, n Werte von 2 bis 4 darstellt, Y die

Gruppe -(C=O)- ist, und R die angegebene Bedeutung hat, $Ar_1$ verschieden von unsubstituiertem Phenyl ist, sowie Salze, insbesondere pharmazeutisch verwendbare, nicht-toxische Säureadditionssalze solcher Verbindungen.

Die Erfindung betrifft insbesondere neue Verbindungen der Formel I, worin R für durch Halogen mit einer Atomnummer bis und mit 35, z.B. Fluor, Chlor oder Brom, mono- oder disubstituiertes oder durch Trifluormethyl, Nitro oder Cyan monosubstituiertes Phenyl steht, wobei Substituenten, die 2- und/oder 3-Stellung einnehmen, $R_1$ Niederalkyl, insbesondere Methyl oder Aethyl, und $R_2$ und $R_3$ jeweils Niederalkyl, in erster Linie Methyl, bedeuten, X für Sauerstoff steht, Alk die Gruppe $-(CH_2)_n-$ darstellt, worin n Werte von 2 bis 3 bedeutet, Y für die Gruppe der Formel -(C=O)- steht, und $Ar_1$ gegebenenfalls durch Halogen mit einer Atomnummer bis und mit 35, z.B. Fluor, substituiertes Phenyl ist, wobei, falls $R_1$, $R_2$ und $R_3$ jeweils Niederalkyl ist, X für Sauerstoff steht, n und R die angegebene Bedeutung hat, $Ar_1$ verschieden von unsubstituiertem Phenyl ist, sowie Salze, insbesondere pharmazeutisch verwendbare, nicht-toxische Säureadditionssalze solcher Verbindungen.

Die Erfindung betrifft insbesondere neue Verbindungen der Formel I, worin R für durch Halogen mit einer Atomnummer bis und mit 35, z.B. Fluor, Chlor oder Brom, mono- oder disubstituiertes, oder vorzugsweise durch Nitro, ferner durch Cyan monosubstituiertes Phenyl steht, wobei Substituenten die 2- und/oder 3-Stellung einnehmen, $R_1$ Niederalkyl, insbesondere Methyl oder Aethyl, und $R_2$ und $R_3$ jeweils Niederalkyl, in erster Linie Methyl bedeuten, X für Sauerstoff steht, Alk die Gruppe $-(CH_2)_n-$ darstellt, worin n die Werte von 2 bis 3 bedeutet, Y für die Gruppe -(C=O)- steht, und $Ar_1$ durch Halogen mit einer Atomnummer bis und mit 35, besonders durch Fluor, in erster Linie in 4-Stellung substituiertes Phenyl ist, sowie Salze, insbesondere pharmazeutisch verwendbare, nicht-toxische Säureadditionssalze solcher Verbindungen.

Die Erfindung betrifft insbesondere die in den Beispielen beschriebenen spezifischen Verbindungen.

Die Verbindungen der Formel I und Salze von solchen Verbindungen mit salzbildenden Eigenschaften können in an sich bekannter Weise hergestellt werden, indem man z.B.

a) eine Verbindung der Formel

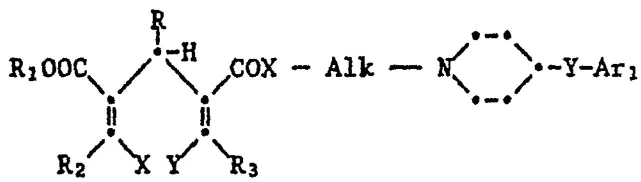

(II),

worin einer der Reste X und Y für die Gruppe der Formel $-NH_2$ steht und der andere Hydroxy oder die Gruppe der Formel $-NH_2$ bedeutet, oder ein Tautomeres davon oder ein entsprechendes Tautomerengemisch ringschliesst, oder

b) eine Verbindung der Formel R-CHO (III) oder ein reaktionsfähiges funktionelles Derivat davon mit einer Verbindung der Formel

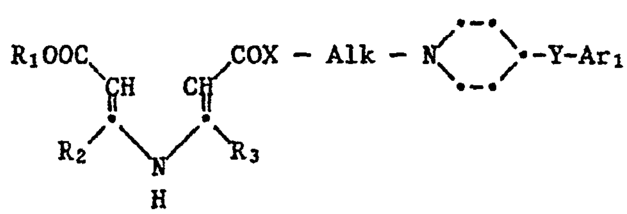

(IV)

oder einem Tautomeren davon oder einem entsprechenden Tautomerengemisch umsetzt, oder

c) in einer Verbindung der Formel

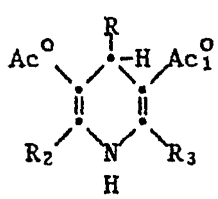

(V),

in welcher einer der Reste $Ac^{o}$ und $Ac_{1}^{o}$ eine in die Gruppe $-COOR_1$ oder in den Rest der Formel

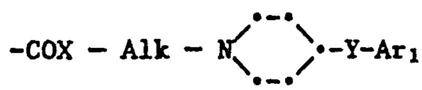 (Va)

überführbare Gruppe bedeutet, und der andere die Gruppe $-COOR_1$ oder die Gruppe der Formel Va bedeutet, den Rest $Ac^{o}$ bzw. $Ac_{1}^{o}$ in die Gruppe $-COOR_1$ bzw. in einen Rest der Formel Va überführt, oder

d) eine Verbindung der Formel

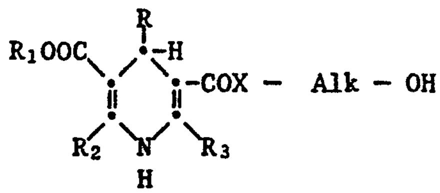 (VI)

oder einen reaktionsfähigen Ester davon, mit einer Verbindung der Formel

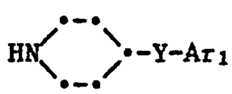 (VIa)

umsetzt, oder

e) eine Verbindung der Formel

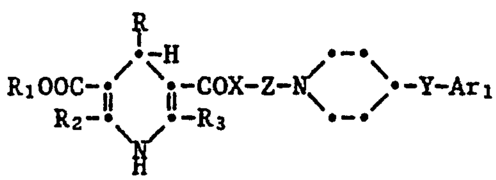 (VII)

worin Z eine mittels Reduktion in die Gruppe Alk überführbare Gruppe darstellt, die Gruppe Z zur Gruppe Alk reduziert, wobei die Ausgangsstoffe der Formel II bis VII sofern sie reaktionsfähige Derivate bilden können oder salzbildende Eigenschaften aufweisen, auch in Form von reaktionsfähigen Derivaten, oder in Form ihrer Salze verwendet werden können, und die Gruppen R, $R_1$, $R_2$, $R_3$, X, Y, $Ar_1$ und Alk die unter der Formel I angegebenen Bedeutungen haben, und, wenn erwünscht, eine erhaltene Verbindung der Formel I in eine

andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I in ein Salz umwandelt, und/oder, wenn erwünscht, ein erhaltenes Isomerengemisch in die individuellen Isomere auftrennt.

Ueblicherweise werden die in der Verfahrensvariante a) verwendeten Ausgangsstoffe der Formel II in situ gebildet, und der verfahrensgemässe Ringschluss kann unter den Reaktionsbedingungen für die Herstellung des Ausgangsmaterials stattfinden. So kann man die Ausgangsstoffe der Formel II und unter den Reaktionsbedingungen üblicherweise auch die entsprechenden Endprodukte der Formel I erhalten, indem man aa) eine Verbindung der Formel III, oder ein reaktionsfähiges funktionelles Derivat davon, mit einer Verbindung der Formel

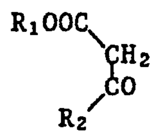

(VIII)

und einer Verbindung der Formel

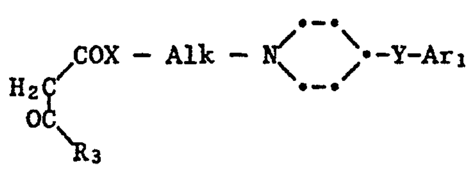

(IX),

und Ammoniak umsetzt, oder ab) eine Verbindung der Formel III oder ein reaktionsfähiges funktionelles Derivat davon mit einer Verbindung der Formel

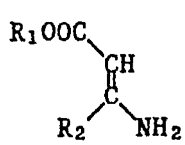

(X),

und einer Verbindung der Formel IX umsetzt, oder ac) eine Verbindung der Formel III oder ein reaktionsfähiges funktionelles Derivat davon mit einer Verbindung der Formel

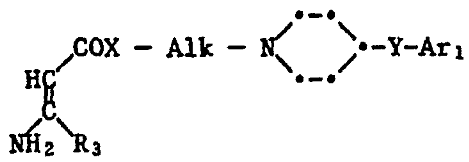

(XI),

und einer Verbindung der Formel VIII oder X umsetzt, oder ad) Ammoniak mit einer Verbindung der Formel

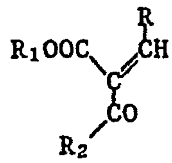

(XII)

und einer Verbindung der Formel IX umsetzt, oder ae) Ammoniak mit einer Verbindung der Formel

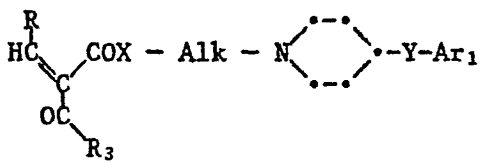

(XIII)

und einer Verbindung der Formel VIII oder X umsetzt, oder af) Ammoniak mit einer Verbindung der Formel

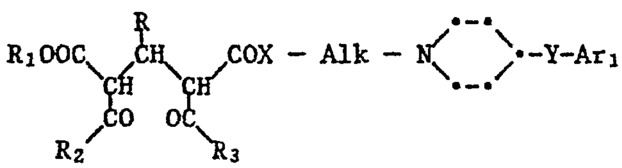

(XIV),

umsetzt, oder ag) eine Verbindung der Formel X mit einer Verbindung der Formel XIII oder der Formel

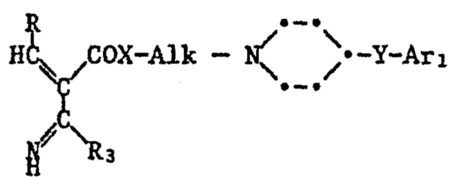

(XV),

umsetzt, oder ah) eine Verbindung der Formel XI mit einer Verbindung der Formel XII oder mit einer Verbindung der Formel

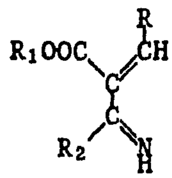

(XVI),

umsetzt. Dabei können mit Ausnahme der Verbindung der Formel III die Verbindungen der Formeln VIII bis XVI in Form von Tautomeren davon oder in Form von Tautomerengemischen verwendet werden; Ausgangsstoffe der obigen Formeln mit salzbildenden Eigenschaften können auch in Form von Salzen verwendet werden. Ferner haben in den obgenannten Verbindungen die Gruppen R, $R_1$, $R_2$, $R_3$, X, Y, $Ar_1$ und Alk die im Zusammenhang mit der Formel I gegebenen Bedeutungen.

Reaktionsfähige funktionelle Derivate des Aldehyds der Formel III sind u.a. die entsprechenden Acetale, d.h. die der Gruppe R entsprechenden Di-(veräthertes Hydroxy)-methyl-Verbindungen, wie Diniederalkyl-, z.B. Dimethyl- oder Diäthylacetale, Acylale, z.B. die entsprechenden Di-Acyloxymethyl- oder Di-Halogen-methyl-Verbindungen, wie Diniederalkanoylacylale, z.B. Diacetylacylale, oder die entsprechenden Dihalogen-, z.B. Dichlor- oder Dibrom-Verbindungen, ferner Additionsverbindungen, wie solche mit einem Alkalimetall-, z.B. Kaliumhydrogensulfit.

Das für die vorstehend beschriebenen Ringschlussreaktionen verwendete Ammoniak kann auch in Form eines, diese Verbindung in situ abgebenden Mittels, z.B. in Form eines Ammoniumsalzes, wie Ammoniumacetat oder Ammoniumhydrogencarbonat, verwendet werden.

Bei der Ringschlussreaktion a), sowie den Kondensationsreaktionen aa) bis ah) zur Herstellung des üblicherweise in situ gebildeten Ausgangsmaterials für die Ringschlussreaktion, handelt es sich um Varianten der Dihydropyridinsynthese von Hantzsch. Bei der Variante aa) werden insgesamt drei Moleküle Wasser abgespalten; bei weiteren Varianten tritt teilweise an die Stelle der Wasserabspaltung eine Additionsreaktion, d.h. die Wasserabspaltung erfolgt schon bei der Herstellung von einem oder von zwei Ausgangsstoffen. Bei der

Umsetzung von Verbindungen der Formel III mit Verbindungen der Formeln XI und X gemäss der Stufe ac), von Verbindungen der Formel X mit Verbindungen der Formel XV gemäss der Stufe ag), oder von Verbindungen der Formel XI mit Verbindungen der Formel XVI gemäss Stufe ah), wird zusätzlich zu bzw. anstelle von Wasser Ammoniak abgespalten. Falls nach der Variante aa) Verbindungen der Formel I hergestellt werden sollen, in denen sich $R_2$ und $R_3$ voneinander unterscheiden, können Nebenprodukte entstehen, die in 2- und 6-Stellung die gleichen Substituenten enthalten. Durch nicht-gleichzeitiges Zusammengeben der Reaktionsteilnehmer kann die Bildung solcher Nebenprodukte indessen herabgesetzt werden, indem man einen bestimmten Reaktionsverlauf fördert, der in situ gemäss einer andern Variante verläuft, da entsprechend der gestaffelten Zugabe der Reaktionskomponenten z.B. zunächst eine Verbindung der allgemeinen Formel X oder der Formel XI entstehen kann.

Die verfahrensgemässen Ringschluss- bzw. Kondensationsreaktionen werden in an sich bekannter Weise durchgeführt, falls notwendig, in Gegenwart eines Kondensationsmittels, insbesondere eines basischen Kondensationsmittels, wie eines Ueberschusses einer basischen Reaktionskomponente oder einer zusätzlichen, z.B. organischen Base, wie Piperidin oder Aethyl-diisopropyl-amin, oder eines Metallalkoholats, wie Alkalimetall-niederalkanolats, und/oder eines geeigneten Dehydratisierungs- oder Wasser-aufnehmenden Mittels, ferner üblicherweise in Gegenwart eines inerten organischen Lösungsmittels und bei Reaktionstemperaturen im Bereich von etwa Raumtemperatur bis etwa 150°C, insbesondere bei Siedetemperatur des Lösungsmittels. Gegebenenfalls erfolgt die Umsetzung in einer Inertgas-, z.B. Stickstoffatmosphäre, und/oder, z.B. bei Verwendung eines niedrigsiedenden Lösungsmittels und/oder von Ammoniak, im geschlossenen Gefäss unter erhöhtem Druck.

Die in den Verfahrensvarianten verwendeten Ausgangsstoffe sind bekannt oder können nach an sich bekannten Verfahren hergestellt werden.

So lassen sich z.B. Ausgangsstoffe der Formel IX durch Umsetzung von Verbindungen der Formel

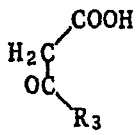

(IXa)

mit Verbindungen der Formel

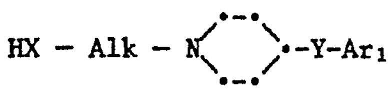

(IXb)

auf übliche Weise herstellen. Anstelle von Carbonsäuren der Formel IXa können auch funktionelle Derivate davon, wie entsprechende Carbonsäureanhydride, insbesondere gemischte Anhydride, wie solche mit Niederalkancarbonsäuren, etwa Ameisensäure, ferner Säurehalogenide, z.B. entsprechende Chloride, Bromide, ferner Säureazide, weiter aktivierte Ester, z.B. Cyanomethylester, verwendet werden. Diese können, gegebenenfalls in Gegenwart von Kondensationsmitteln, durch Umsetzung mit einer Verbindung der Formel IXb, freie Carbonsäuren der Formel IXa auch durch Umsetzen mit Verbindungen der Formel IXb, worin anstelle der Gruppe HX- die Azidogruppe steht, zu Verbindungen der Formel IX umgesetzt werden. Carbonsäuren der Formel IXa können auch als Salze, insbesondere als Alkalimetall- oder Erdalkalimetallsalze mit reaktionsfähigen Estern von Alkoholen der Formel IXb, worin X für Sauerstoff steht, wie entsprechenden Halogeniden, z.B. Chloriden, Bromiden oder Jodiden, oder organischen Sulfonsäureestern, z.B. Niederalkansulfonsäure- oder Arensulfonsäureestern, wie Methansulfonsäure- bzw. p-Toluolsulfonsäureestern, zu entsprechenden Carbonsäureestern umgesetzt werden, oder man hydrolysiert entsprechende hydrolysierbare Iminoester, wie entsprechende Iminoniederalkylester, zu den Estern. Iminoester dieser Art sind beispielsweise aus den Verbindungen der Formel IXa entsprechenden Nitrilen der Formel

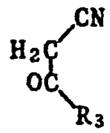

(IXc)

durch Umsetzung mit Verbindungen der Formel IXb, worin X für Sauerstoff steht, in Gegenwart eines sauren Kondensationsmittels, etwa Chlorwasserstoff, in einem geeigneten Lösungsmittel, etwa eines solchen inerten Charakters, wie eines Aromaten, z.B. Benzol, auf übliche Weise zugänglich.

Verbindungen der Formel IXb wiederum sind auf an sich bekannte Weise zugänglich, z.B. durch Umsetzung von Verbindungen der Formel

HX-Alk-A (IXd),

worin A eine geeignete Abgangsgruppe, z.B. eine reaktionsfähige veresterte Hydroxygruppe, wie etwa Halogen, z.B. Chlor, Brom oder Jod oder eine Sulfonyloxygruppe, z.B. eine Arylsulfonyloxy-, wie p-Toluolsulfonyloxygruppe, darstellt, mit Verbindungen der Formel

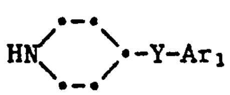

(IXe),

zweckmässigerweise in Gegenwart eines basischen Kondensationsmittels, wie eines Oxids, Hydroxids oder Carbonats eines Alkalimetall- oder Erdalkalimetalls, wie Natriumhydroxid oder Calciumcarbonat, üblicherweise in Gegenwart eines Lösungsmittels, etwa eines Niederalkanols, wie Aethanol, und bei erhöhter oder erniedrigter Temperatur. Verbindungen der Formel IXe können auch in Form ihrer Metallderivate eingesetzt werden, worin das am Stickstoff stehende Wasserstoffatom durch ein geeignetes Metall, etwa Lithium oder Kalium ersetzt ist. In solchen Fällen erfolgt die beschriebene Umsetzung mit Verbindungen der Formel IXd in einem inerten wasserfreien Lösungsmittel, etwa eines solchen ätherartigen Charakters, wie Tetrahydrofuran, oder eines aromatischen Lösungsmittels, etwa Toluol.

Der Formel IXe entsprechende Metallverbindungen sind in üblicher Weise, z.B. durch Umsetzung mit einer geeigneten Alkalimetallorganischen Verbindung, etwa Butyllithium, in einem wasserfreien Lösungsmittel, wie Tetrahydrofuran, zweckmässigerweise unter einem Schutzgas, z.B. Argon, zugänglich, wobei die im Reaktionsgemisch enthaltene Metallverbindung, ohne diese zu isolieren, für die oben beschriebene Umsetzung verwendet werden kann.

Verbindungen der Formel IXe wiederum sind auf an sich bekannte Weise herstellbar, etwa aus solchen Ausgangsstoffen, die anstelle des Piperidinringes eine geeignete, in einen Piperidinring überführbare Gruppe enthält. Solche Gruppen sind etwa solche, die z.B. mittels Reduktion oder mittels ringschliessender Kondensation den Piperidinring bilden, etwa ein entsprechendes 2-Piperidon, welches mittels eines geeigneten Reduktionsmittels, wie Lithiumaluminiumhydrid, in einen Ausgangsstoff der Formel IXe umgewandelt werden kann. Oder man geht z.B. von einem entsprechenden 1,5-Di-halogenpentan, etwa 1,5-Dibrompentan aus, welches mittels Ammoniak, gegebenenfalls im geschlossenen Gefäss und unter Druck, einen Ausgangsstoff der Formel IXe ergibt.

Die Umsetzung von freien Carbonsäuren der Formel IXa mit Verbindungen der Formel IXb erfolgt vorteilhaft in Gegenwart eines sauren, die Wasserabspaltung fördernden Katalysators, wie einer Protonensäure, z.B. Chlorwasserstoff-, Bromwasserstoff-, Schwefel-, Phosphor- oder Borsäure, Benzolsulfon- oder Toluolsulfonsäure, oder einer Lewissäure, z.B. von Bortrifluorid-Aetherat, in einem Ueberschuss des eingesetzten Alkohols und/oder in einem inerten Lösungsmittel, erforderlichenfalls unter destillativer, z.B. azeotroper, Entfernung des bei der Reaktion freigesetzten Wassers. Weiter kann man die Umsetzungen auch in Gegenwart von wasserbindenden Kondensationsmitteln, wie geeignet substituierten Carbodiimiden, z.B. N,N'-Diäthyl-, N,N'-Dicyclohexyl-oder N-Aethyl-N'-(3-dimethylaminopropyl)-carbodiimid, in inerten organischen Lösungsmitteln durchführen. Gemischte Anhydride, insbesondere Säurehalogenide, werden

beispielsweise in Gegenwart säurebindender Mittel, z.B. organischer, insbesondere tertiärer Stickstoffbasen, wie Triäthylamin, Aethyldiisopropylamin oder Pyridin, oder auch anorganischer Basen, z.B. Alkalimetall- oder Erdalkalimetallhydroxiden oder -carbonaten, wie Natrium-, Kalium-oder Calciumhydroxid bzw. -carbonat, mit Alkoholen oder mit Alkoholaten, z.B. Alkalimetall-niederalkanolaten, umgesetzt.

Die Umsetzungen von reaktionsfähigen Estern, z.B. Cyanmethyl- oder Pentachlorphenylestern, mit Verbindungen der Formel IXb werden beispielsweise in einem gegenüber den Reaktionsteilnehmern inerten Lösungsmittel im Temperaturbereich von etwa 0°C bis etwa 120°C, vorzugsweise bei Raumtemperatur bis etwa 60°C, durchgeführt.

Die Hydrolyse von Imidoesterausgangsstoffen erfolgt beispielsweise mittels wasserhaltiger Mineralsäuren, wie Salzsäure oder Schwefelsäure, wobei man z.B. die bei der Addition von Chlorwasserstoff an Nitrile und Umsetzung mit wasserfreien Alkoholen, insbesondere unsubstituierten oder substituierten Niederalkanolen, erhaltenen Iminoester-salze, z.B. -hydrochloride, nach Zusatz von Wasser direkt zu den entsprechenden Estern hydrolysieren kann. Man kann z.B. auch aus einem Gemisch von Nitril, Alkohol und Schwefelsäure mit geeignetem Wassergehalt ohne Isolierung des in situ entstandenen Imidoesters die gewünschte Esterverbindung der Formel IX erhalten.

Ausgangsstoffe der Formeln XI, XIII, XIV und XV können ausgehend von entsprechenden Ausgangsmaterialien auf analoge und übliche Weise hergestellt werden.

Ausgangsstoffe der Formel IV werden analog wie bei der Herstellung von Ausgangsstoffen der Formel II beschrieben in situ gebildet, und der verfahrensgemässe Ringschluss zu den Endprodukten der Formel I kann unter den für die Herstellung des Ausgangsmaterials gegebenen Bedingungen im gleichen Reaktionsansatz erfolgen. Dementsprechend sind Ausgangsstoffe der Formel IV durch Umsetzung von Verbindungen der Formel IX mit solchen der Formel VIII und Ammoniak, oder von

Verbindungen der Formel IX mit solchen der Formel X, oder von Verbindungen der Formel XI mit solchen der Formel VIII oder X zugänglich, wobei solche Umsetzungen üblicherweise in einem geeigneten Lösungsmittel, etwa einem Niederalkanol, wie Aethanol, gegebenenfalls bei erhöhter oder erniedrigter Temperatur, zweckmässigerweise unter einem Schutzgas, wie Stickstoff, durchgeführt werden.

Ausgangsstoffe der Formel V können entsprechend dem bzw. den in ihnen enthaltenen Rest(en) $Ac^o$ und/oder $Ac_1^o$, beispielsweise Carbonsäuren ($Ac^o$ und/oder $Ac_1^o$ ist Carboxyl), Carbonsäureanhydride, insbesondere gemischte Anhydride, wie Säurehalogenide, z.B. -chloride oder -bromide, oder Azide ($Ac^o$und/oder $Ac_1^o$ ist Halogencarbonyl, z.B. Chlor-oder Bromcarbonyl oder Azidocarbonyl), weiter aktivierte Ester, z.B. Cyanmethylester ($Ac^o$ und/oder $Ac_1^o$ ist Cyanmethoxycarbonyl) sein. Diese können, gegebenenfalls in Gegenwart von Kondensationsmitteln, durch Behandeln mit einem entsprechenden Alkohol, in die entsprechenden Ester umgewandelt werden, beispielsweise zur Umwandlung der Gruppe $Ac^o$ in eine der Gruppe $-COOR_1$ entsprechende Carboxylestergruppe durch Umsetzung mit einem der Bedeutung von $R_1$ entsprechenden unsubstituierten oder substituierten Niederalkanol, oder einem reaktionsfähigen Derivat davon, z.B. einem entsprechenden Alkoholat, freie Carbonsäuren auch durch Umsetzen mit geeigneten Diazo-Verbindungen, wie unsubstituierten oder substituierten Diazoniederalkanen, wobei Verbindungen der Formel I entstehen, welche die Gruppe $-COOR_1$ enthalten.

Zur Umwandlung der Gruppe $Ac_1^o$ in die Gruppe der oben definierten Formel Va setzt man einen Ausgangsstoff der Formel V mit einer Verbindung der Formel IXb in üblicher Weise um. Carbonsäureester der angegebenen Art, worin X für Sauerstoff steht, können ebenfalls erhalten werden, wenn als Ausgangsstoffe Salze, insbesondere Alkalimetall- oder Erdalkalimetallsalze, der freien Carbonsäuren verwendet und diese mit reaktionsfähigen Estern von, den Verbindungen der Formel IXb entsprechenden Alkoholen, worin X Sauerstoff ist, wie entsprechenden Halogeniden, z.B. Chloriden, Bromiden oder

Iodiden, oder organischen Sulfonsäureestern, z.B. Niederalkansulfonsäure-oder Arensulfonsäureestern, wie Methansulfonsäure- bzw. p-Toluolsulfonsäureestern, behandelt werden, oder wenn man entsprechende hydrolysierbare Iminoester, wie entsprechende Iminoniederalkylester, zu den Estern hydrolysiert.

Iminoester dieser Art sind z.B. aus Ausgangsstoffen der Formel V, worin $Ac^o$ und/oder $Ac_1^o$ die Cyangruppe darstellt, durch Umsetzung mit einem der Bedeutung von $R_1$ entsprechenden Niederalkanol und/oder einem Alkohol der Formel IXb, worin X Sauerstoff ist, in Gegenwart eines sauren Kondensationsmittels, wie Chlorwasserstoff oder konz. Schwefelsäure zugänglich.

Die Umsetzung von freien Carbonsäuren mit Alkoholen, wie unsubstituierten oder substituierten Niederalkanolen oder mit Verbindungen der Formel IXb, erfolgt vorteilhaft in Gegenwart eines sauren, die Wasserabspaltung fördernden Katalysators, wie einer Protonensäure, z.B. Chlorwasserstoff-, Bromwasserstoff-, Schwefel-, Phosphor- oder Borsäure, Benzolsulfon- oder Toluolsulfonsäure, oder einer Lewissäure, z.B. von Bortrifluorid-Aetherat, in einem Ueberschuss des eingesetzten Alkohols bzw. der Verbindung der Formel IXb und/oder in einem inerten Lösungsmittel, erforderlichenfalls unter destillativer, z.B. azeotroper, Entfernung des bei der Reaktion freigesetzten Wassers. Weiter kann man die Umsetzungen auch in Gegenwart von wasserbindenden Kondensationsmitteln, wie geeignet substituierten Carbodiimiden, z.B. N,N'-Diäthyl-, N,N'-Dicyclohexyl- oder N-Aethyl-N'-(3-dimethylaminopropyl)-carbodiimid, üblicherweise in inerten organischen Lösungsmitteln durchführen. Gemischte Anhydride, insbesondere Säurehalogenide, werden beispielsweise in Gegenwart säurebindender Mittel, z.B. organischer, insbesondere tertiärer Stickstoffbasen, wie Triäthylamin, Aethyldiisopropylamin oder Pyridin, oder auch anorganischen Basen, z.B. Alkalimetall- oder Erdalkalimetallhydroxiden oder -carbonaten, wie Natrium-, Kalium- oder Calciumhydroxid bzw. -carbonat, mit Alkoholen oder mit Alkoholaten, z.B. Alkalimetall-niederalkanolaten, umgesetzt.

Die Umsetzungen von der Formel V entsprechenden reaktionsfähigen Estern, z.B. Cyanmethyl-, Benztriazol-1-yl oder Pentachlorphenylestern, mit der Bedeutung von $R_1$ entsprechenden Niederalkanolen bzw. mit Verbindungen der Formel IXb werden beispielsweise in einem gegenüber den Reaktionsteilnehmern inerten Lösungsmittel im Temperaturbereich von etwa 0°C bis etwa 120°C, vorzugsweise bei Raumtemperatur bis etwa 60°C, durchgeführt.

Die Hydrolyse von Imidoesterausgangsstoffen, erfolgt beispielsweise mittels wasserhaltiger Mineralsäuren, wie Salzsäure oder Schwefelsäure, wobei man z.B. die bei der Addition von Chlorwasserstoff an Nitrile und Umsetzung mit wasserfreien Alkoholen, insbesondere unsubstituierten oder substituierten Niederalkanolen, erhaltenen Iminoester-salze, z.B. -hydrochloride, nach Zusatz von Wasser direkt zu den entsprechenden Estern hydrolysieren kann. Man kann Ester z.B. auch aus einem Gemisch von Nitril, Alkohol und Schwefelsäure mit geeignetem Wassergehalt ohne Isolierung des <u>in situ</u> entstandenen Imidoesters die gewünschte Esterverbindung der Formel I erhalten.

Ausgangsstoffe der Formel V mit freier Carboxylgruppe $Ac^o$ und/oder $Ac_1^o$ können z.B. erhalten werden, indem man den entsprechenden 2-Cyanäthylester herstellt, wobei man z.B. in einem der vorstehend beschriebenen Verfahren ab) oder ag) eine Verbindung der Formel X einsetzt, in der anstelle der Gruppe $-COOR_1$ eine 2-Cyanäthoxycarbonylgruppe steht; z.B. kann man einen, die Gruppe $R_2$ enthaltenden 3-Aminocrotonsäure-2-cyanäthylester mit den übrigen Reaktionskomponenten umsetzen, und anschliessend die so erhaltene 2-Cyanäthylester-Verbindung unter milden Bedingungen, z.B. mittels wässrigem oder wässerig-niederalkanolischem 1-n. Natriumhydroxid bei Raumtemperatur, zur freien Carbonsäure spalten. Letztere kann, falls notwendig, in an sich bekannter Weise in die gewünschten reaktionsfähigen funktionellen Derivate übergeführt werden.

Die als Ausgangsstoffe für die Verfahrensvariante c) ebenfalls in Betracht kommenden Nitrilverbindungen der Formel V können z.B. analog zu einer der Verfahrensvarianten aa) bis ah) hergestellt

werden, indem man Ausgangsstoffe verwendet, die anstelle des Restes $-COOR_1$ bzw. der Gruppe der Formel IXb eine Cyangruppe enthalten, wie beispielsweise anstelle einer Verbindung der Formel X ein, die Gruppe $R_2$ enthaltendes 3-Aminocrotonitril.

Die für die Verfahrensvariante d) benötigten Ausgangsstoffe der Formel VI können auf an sich bekannte Weise hergestellt werden, z.B. analog den in den Verfahrensstufen aa) bis ah) beschriebenen Umsetzungen, wobei anstelle der Verbindungen IX, XI, XIII, XIV oder XV solche Ausgangsmaterialien eingesetzt werden, die anstelle der Gruppe der Formel

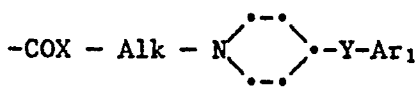 (VIa),

die Gruppe der Formel -COX-Alk-OH (VIb) bzw. einen reaktionsfähigen Ester davon aufweisen. Reaktionsfähige Ester sind z.B. solche mit einer Halogenwasserstoffsäure, z.B. Salzsäure, oder einer organischen Sulfonsäure, wie einer Arylsulfonsäure, z.B. p-Toluolsulfonsäure, gebildete Ester, in denen die Hydroxygruppe z.B. durch Halogen, wie Chlor oder Brom, oder z.B. durch Arylsulfonyloxy, wie p-Toluolsulfonyloxy ersetzt ist. So kann z.B. ein Ausgangsstoff der Formel

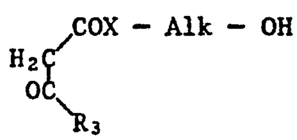 (VIc)

analog den für die Herstellung von Ausgangsstoffen der Formel IX beschriebenen Methoden, z.B. durch Umsetzung von Verbindungen der Formel IXa mit Verbindungen der Formel HX-Alk-OH (VIa) oder einem reaktionsfähigen Ester, etwa einem Halogenid, wie Bromid oder Jodid, wie beschrieben, erhalten werden. Oder man setzt ein Nitril der Formel IXc mit einer Verbindung der Formel VId, worin X Sauerstoff ist, in Gegenwart eines sauren Kondensationsmittels, etwa Chlorwasserstoff, zum entsprechenden Iminoester-Salz um, das dann mittels Wasser im sauren Medium, z.B. wie oben beschrieben, zum entsprechenden Carbonsäureester hydrolysiert wird.

Die obigen Reaktionen und ebenso die Verfahrensvariante d) können unter an sich bekannten Reaktionsbedingungen durchgeführt werden, in Ab- oder üblicherweise Anwesenheit von Lösungs- oder Verdünnungsmitteln, je nach Art der Reaktion und/oder Reaktionsteilnehmer bei erniedrigter oder erhöhter Temperatur, z.B. im Temperaturbereich von etwa -10°C bis etwa 150°C, unter atmosphärischem Druck oder in einem geschlossenen Gefäss, gegebenenfalls unter Druck, und/oder in einer inerten Atmosphäre, z.B. unter einer Stickstoffatmosphäre.

Die im Ausgangsmaterial der Formel VII der Verfahrensvariante e) vorhandenen, mittels Reduktion in die Gruppe Alk überführbaren Gruppen Z enthalten Doppel- und/oder Dreifachbindungen und/oder Carbonyl- bzw. Thiocarbonylgruppen im entsprechenden Alkylenrest.

Die Reduktion von ungesättigten Gruppen zur Kohlenstoff-Kohlenstoff-Einfachbindung erfolgt beispielsweise mittels aktiviertem Wasserstoff, wie Wasserstoff in Gegenwart eines Hydrierkatalysators, z.B. eines Nickel-, Platin- oder Palladiumkatalysators, während die Reduktion von Carbonylgruppen zur Methylengruppe mittels Wasserstoff in Gegenwart z.B. eines Kupferchromit-Katalysators oder nach der Methode von Clemmensen, z.B. mittels gegebenenfalls analgamiertem Zink in einer Mineralsäure, etwa Salzsäure, erfolgen kann. Ist die zu reduzierende Carbonylgruppe an ein Stickstoffatom gebunden und liegt somit ein Carboxamid vor, so kann die Reduktion zur Methylengruppe in üblicher Weise, z.B. mit einem Metallhydrid, etwa Lithiumaluminiumhydrid erfolgen. Die Reduktion von Thiocarbonylgruppen kann auf ähnliche Weise erfolgen, z.B. unter Verwendung eines schwefelfesten Katalysators. Die Reduktion der vorstehend genannten Gruppen kann ferner mittels eines Hydridreduktionsmittels, z.B. Natriumborhydrid oder Diboran, erfolgen. Bei diesen Reduktionen muss, sofern erwünscht, Sorge dafür getragen werden, dass andere gegen Reduktion empfindliche Gruppen, z.B. ungesättigte Gruppen oder

Nitrogruppen, nicht angegriffen werden, z.B. durch Auswahl des geeigneten Reduktionsmittels, dessen für die durchzuführende Reduktion erforderliche Dosierung und/oder geeigneter Verfahrensbedingungen, z.B. erhöhte oder erniedrigte Temperatur und/oder durch Anwendung eines geeigneten Lösungsmittels. Diese Umsetzungen werden in an sich bekannter Weise durchgeführt, üblicherweise in Anwesenheit von Lösungs- und Verdünnungsmitteln, je nach Art der Reaktion und/oder Reaktionsteilnehmer bei erniedrigter oder erhöhter Temperatur, z.B. im Temperaturbereich von etwa -15°C bis etwa 120°C unter normalem Druck oder im geschlossenen Gefäss, gegebenenfalls unter Druck und/oder unter einem Schutzgas, etwa Stickstoff.

Ausgangsstoffe der Formel VII können, sofern sie neu sind, nach an sich bekannten Methoden hergestellt werden, z.B. analog den unter a) bis d) beschriebenen Verfahren. So kann man z.B. analog den in den Stufen aa) bis ah) beschriebenen Reaktionsschritten verfahren, wenn man anstelle der Verbindungen IX, XI, XIII, XIV oder XV solche Verbindungen einsetzt, die anstelle der oben definierten Gruppe Va die Gruppe der Formel

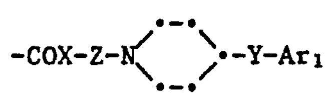

(VIIa)

enthält.

So kann z.B. ein Ausgangsstoff der Formel

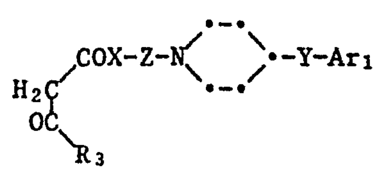

(VIIb),

analog den für die Herstellung von Verbindungen der Formel IX beschriebenen Methoden, etwa durch Umsetzung von Verbindungen der Formel IXa oder reaktionsfähigen Derivaten davon, z.B. Anhydriden, Säurehalogeniden, etwa Chloriden, Aziden, gemischten Estern wie Cyanomethylestern oder Pentachlorphenolestern, verwendet werden.

Carbonsäuren der Formel IXa können auch als Salze, insbesondere als Alkalimetall- oder Erdalkalimetallsalze mit reaktionsfähigen Estern der Formel

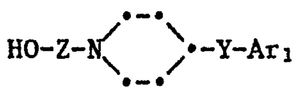

(VIIc),

wie entsprechenden Halogeniden, z.B. Chloriden, Bromiden oder Jodiden, oder organischen Sulfonsäureestern, etwa die mit Niederalkansulfonsäuren, wie Methansulfonsäure, oder Arylsulfonsäuren, wie Benzolsulfonsäure, zu entsprechenden Carbonsäureestern umgesetzt werden. Alkohole der Formel VIIc können andererseits mit Nitrilen der Formel IXc in üblicher Weise z.B. in Gegenwart saurer Kondensationsmittel, wie einer Mineralsäure etwa Chlorwasserstoff- oder Schwefelsäure, zu den Salzen entsprechender Iminoester umgesetzt werden, die dann mittels Wasser, wie beschrieben, zu den Carbonsäureestern hydrolysiert werden. Diese Umsetzungen werden unter üblichen und an sich bekannten Reaktionsbedingungen vorgenommen. Ausgangsstoffe der Formel VIIc wiederum, sind, soweit sie neu sind, auf übliche Weise, z.B. durch Umsetzung von Verbindungen der Formel HO-Z-A (VIId), worin A eine geeignete Abgangsgruppe, z.B. Halogen, wie Chlor, Brom oder Jod, oder eine Sulfonyloxy-, wie p-Toluolsulfonyloxygruppe ist, mit Verbindungen der Formel IXe auf übliche Weise zugänglich.

Verfahrensgemäss erhältliche Verbindungen der Formel I können in an sich bekannter Weise in andere Verbindungen der Formel I umgewandelt werden, etwa durch Umwandlung von in Verbindungen der Formel I enthaltenen Substituenten in andere von der Formel I umfasste Substituenten.

So kann man z.B. eine veresterte Carboxygruppe $-COOR_1$ durch Umesterung in eine andere Estergruppe überführen. Dabei verwendet man als Reagens vorzugsweise Alkoholverbindungen, die einen Siedepunkt aufweisen, der deutlich über demjenigen des Alkohols der veresterten Gruppe in der umzuwandelnden Verbindung der Formel I liegt, und führt die Reaktion z.B. in einem Ueberschuss der Alkoholverbindung und/oder einem inerten organischen, vorzugsweise ebenfalls deutlich über dem Alkohol der veresterten Gruppe siedenden Lösungsmittel, vorzugsweise in Gegenwart eines Katalysators, z.B. eines Alkalimetall-niederalkanolats, wie Natrium- oder Kaliummethanolat oder -äthanolat, in der Wärme und üblicherweise unter Abdestillieren des freigesetzten Alkohols durch.

Verbindungen der Formel I, worin Y für die Gruppe -(C=O)- steht, kann man in üblicher Weise mittels Reduktion,in Verbindungen der Formel I umwandeln, worin Y die Gruppe -CHOH- darstellt, wobei gegebenenfalls vorhandene ungesättigte Gruppen in gesättigte, bzw. Dreifachbindungen erhaltende Gruppen in solche mit Zweifachbindungen umgewandelt werden können.

Für die Reduktion kann man z.B. katalytisch aktivierten Wasserstoff, etwa Wasserstoff in Gegenwart eines Hydrierkatalysators, wie eines Edelmetallkatalysators, z.B. Platin oder Palladium, ferner Raney-Nickel verwenden. Ebenso kann ein geeignetes Hydridreduktionsmittel, etwa Diboran oder Natriumborhydrid, ferner Lithiumaluminiumhydrid, in einem geeigneten Lösungsmittel, etwa eines solchen ätherartigen Charakters, wie Tetrahydrofuran, eingesetzt werden.

Ferner kann man Verbindungen der Formel I, in welchen R und/oder $Ar_1$ geeignete azaheterocyclische Gruppen darstellen, deren Ringstickstoffatome N-oxidiert werden können, in entsprechende N-Oxide umwandeln. Die Oxidation kann in an sich bekannter Weise durchgeführt werden, z.B. durch Behandeln mit organischen Persäuren, wie Niederalkanpersäuren oder Arenpersäuren, wie gegebenenfalls geeignet substituierten Perbenzoesäuren, z.B. Peressig- oder 3-Chlorperbenzoesäure, vorzugsweise bei Zimmertemperatur oder etwas darüber liegender Reaktionstemperatur, oder mit wässrigem Wasserstoffperoxid, z.B. bei Temperaturen von bis zu 100°C, in Gegenwart oder Abwesenheit von Niederalkansäuren, z.B. Essigsäure. Dabei muss, insbesondere bei Verwendung von Persäuren, darauf geachtet werden, dass keine Ueberoxidation aufgrund einer zu langen Reaktionsdauer eintreten kann.

Je nach Reaktionsbedingungen können Verbindungen der Formel I freier Form oder in Form von Salzen erhalten werden.

So können erhaltene Säureadditionssalze in an sich bekannter Weise, z.B. durch Behandeln mit einer Base, wie einem Alkalimetallhydroxid, in die freien Verbindungen, oder z.B. durch Behandeln mit geeigneten Säuren oder Derivaten davon in andere Salze umgewandelt werden. Erhaltene freie Verbindungen der Formel I können, z.B. durch Behandeln mit Säuren oder entsprechenden Anionenaustauschern, in ihre Salze umgewandelt werden.

Infolge der engen Beziehung zwischen den Verbindungen der Formel I in freier Form und in Form von Salzen, sind im Vorausgegangenen und nachfolgend unter den freien Verbindungen oder ihren Salzen sinn- und zweckgemäss gegebenenfalls auch die entsprechenden Salze bzw. freien Verbindungen zu verstehen.

Die Verbindungen der Formel I, einschliesslich deren Salze können auch in Form ihrer Hydrate erhalten werden, oder ihre Kristalle können z.B. das zur Kristallisation verwendete Lösungsmittel einschliessen.

Verbindungen der Formel I können, je nach chemischer Struktur, Verfahrensreaktion und/oder Art der Ausgangsstoffe, in Form von Racematgemischen, Racematen oder optischen Antipoden erhalten werden.

Erhaltene Racematgemische können auf Grund der physikalisch-chemischen Unterschiede der Racemate in bekannter Weise in die reinen Racemate bzw. Diastereomeren aufgetrennt werden, beispielsweise durch Chromatographie und/oder fraktionierte Kristallisation.

Racemate lassen sich nach an sich bekannten Methoden in die optischen Antipoden zerlegen, beispielsweise durch Umkristallisation aus einem optisch aktiven Lösungsmittel, mit Hilfe von geeigneten Mikroorganismen oder durch Umsetzen einer Verbindung der Formel I

mit salz-bildenden, z.B. basischen, Eigenschaften mit einem optisch aktiven, salzbildenden Mittel, wie einer optisch aktiven Säure, und Trennen der auf diese Weise erhaltenen Gemische von Salzen, z.B. auf Grund ihrer verschiedenen Löslichkeiten, in die diastereomeren Salze, aus denen die Antipoden, z.B. durch Behandeln mit einer Base, freigesetzt werden können.

Optische Antipoden von neutralen Verbindungen der Formel I kann man z.B. auch gemäss Verfahren c) unter Verwendung einer optisch aktiven Säure der Formel V ($Ac^o$ bedeutet Carboxy, $Ac_1^o$ ist verestertes oder amidiertes Carboxy) erhalten, wobei man diese z.B. aus der entsprechenden racemischen Säure in üblicher Weise, z.B. durch Salzbildung mit einer optisch aktiven Base, Trennung der diastereomeren Salze und Freisetzung der optisch aktiven Säure und deren Umwandlung in eine der Formel I entsprechende, die Gruppe $-COOR_1$ enthaltende Verbindung erhält.

Ferner kann man z.B. Verbindungen der Formel I, mit der Gruppe $-COOR_1$ unter Verwendung eines optisch aktiven Alkohols nach dem oben beschriebenen Verfahren umestern und das so erhältliche Diastereomerengemisch, z.B. mittels fraktionierter Kristallisation, in die Antipoden auftrennen.

Vorteilhafterweise isoliert man aus einem Diastereomerengemisch bzw. Racemat das pharmakologisch aktivere Diastereomere bzw. den aktiveren Antipoden.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivats, z.B. Salzes, und/oder seiner Racemate bzw. Antipoden verwendet oder unter den Reaktionsbedingungen bildet. Salze von Ausgangsstoffen mit salzbildenden basischen Eigenschaften sind z.B. solche mit Mineralsäuren, wie Salzsäure, Schwefelsäure oder Phosphorsäure, oder mit organischen Säuren, z.B. Essigsäure.

Bei den Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Zwischenprodukte sowie Verfahren zur deren Herstellung bilden ebenfalls einen Gegenstand der vorliegenden Erfindung.

Die Erfindung betrifft ebenfalls die Verwendung der Verbindungen der Formel I oder von pharmazeutisch verwendbaren Salzen von solchen Verbindungen mit salzbildenden Eigenschaften, insbesondere als pharmakologisch, in erster Linie als Coronardilatatoren und Antihypertensiva zur Behandlung von cardiovasculären Krankheitszuständen, wie Angina pectoris und ihre Folgen, Gefässspasmen, hohem Blutdruck und Herzinsuffizienz wirksame Verbindungen. Dabei kann man sie, vorzugsweise in Form von pharmazeutischen Präparaten, in einem Verfahren zur prophylaktischen und/oder therapeutischen Behandlung des tierischen und menschlichen Körpers, insbesondere zur Behandlung von cardiovasculären Krankheitszuständen, wie Angina pectoris und ihre Folgen, Gefässspasmen, hohem Blutdruck und Herzinsuffizienz verwenden.

Die Dosierung des Wirkstoffs, der allein oder zusammen mit dem üblichen Träger- und Hilfsmaterial verabreicht wird, hängt von der zu behandelnden Spezies, deren Alter und individuellem Zustand, sowie der Verabreichungsweise ab. Die täglichen Dosen liegen z.B. für Mammalien mit einem Körpergewicht von etwa 70 kg, je nach Art der Erkrankung, individuellem Zustand und Alter, vorzugsweise zwischen etwa 10 und 500 mg, insbesondere zwischen etwa 50 mg und etwa 250 mg, und speziell etwa bei 70 mg bis 150 mg bei oraler Verabreichung.

Die Erfindung betrifft im weiteren pharmazeutische Präparate, die Verbindungen der Formel I oder pharmazeutisch verwendbare Salze von solchen Verbindungen mit salzbildenden Eigenschaften als Wirkstoffe enthalten, Verfahren zu ihrer Herstellung, sowie die Verwendung von Verbindungen der Formel I oder deren pharmazeutisch verwendbaren

Salze zur Herstellung von Arzneimitteln als Coronardilatoren und Antihypertensiva zur Behandlung von cardiovasculären Krankheitszuständen, wie Angina pectoris und ihre Folgen, Gefässspasmen, zentrale und periphere Durchblutungsstörungen, hohem Blutdruck, Arrythmien und Herzinsuffizienz, ferner zur Anwendung als Hemmer der Plättchenaggregation.

Bei den erfindungsgemässen pharmazeutischen Präparaten handelt es sich um solche zur enteralen, wie peroralen oder rektalen, weiter zur sublingualen, sowie zur parenteralen Verabreichung an Warmblüter. Entsprechende Dosiseinheitsformen, insbesondere zur peroralen und/oder sublingualen Verabreichung, z.B. Dragées, Tabletten oder Kapseln, enthalten vorzugsweise von etwa 10 mg bis etwa 300 mg, insbesondere von etwa 20 mg bis etwa 200 mg einer Verbindung der Formel I oder eines pharmazeutisch annehmbaren Salzes einer zur Salzbildung befähigten entsprechenden Verbindung zusammen mit pharmazeutisch verwendbaren Trägerstoffen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose und/oder, wenn erwünscht, Sprengmittel, wie die obgenannten Stärken, ferner Carboxymethylstärke, quervernetztes Polyvinyl-pyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyäthylenglykol und/oder Titandioxid enthalten, oder Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Lactose, Bindemitteln, wie Stärken und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können. Bevorzugt sind u.a. Kapseln, die sowohl leicht zerbissen werden können, um durch sublinguale Aufnahme des Wirkstoffes eine möglichst rasche Wirkung zu erzielen, als auch unzerkaut geschluckt werden können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffes mit einer Grundmasse enthalten; als Grundmassenstoffe kommen z.B. flüssige Triglyceride, Polyäthylenglykole oder Polyäthylenglykole oder Paraffinkohlenwasserstoffe in Frage.

Zur parenteralen Verabreichung eignen sich in erster Linie wässrige Lösungen eines Wirkstoffes in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat, oder Triglyceride verwendet, oder wässrige Injektionssuspensionen, welche viskositätserhöhende Stoffe, z.B. Natriumcarboxymethylcellulose, Sorbit und/oder Dextran und gegebenenfalls Stabilisatoren enthalten.

Die pharmazeutischen Präparate der vorliegenden Erfindung können in an sich bekannter Weise, z.B. mittels konventioneller Misch-, Granulier-, Dragier-, Lösungs- oder Lyophilisierungsverfahren hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Beispiel 1: Eine Lösung von 24 g 2,6-Dimethyl-4-(3-nitrophenyl)-1,4-dihydropyridin-3,5-dicarbonsäure-3-(1-benzotriazolyl)-ester-5-methylester in 60 ml Dimethylformamid wird in einer Argonatmosphäre nacheinander mit 12,1 ml N-Aethylmorpholin und 13 g 2-[4-(p-Fluorbenzoyl)-piperidino]-äthylaminhydrochlorid versetzt und die Suspension 1 Stunde bei Eiskühlung, dann 1 Stunde bei 20° und schliesslich 17 Stunden bei 60° Badtemperatur gerührt. Anschliessend wird das Reaktionsgemisch am Rotationsverdampfer unter Hochvakuum vom Lösungsmittel befreit und der Rückstand in 150 ml Essigsäureäthylester und 50 ml Wasser gelöst. Die organische Phase wird über Natriumsulfat getrocknet, eingedampft und das erhaltene Rohprodukt über 1 kg Kieselgel unter Verwendung von Methylenchlorid: Methanol (95:5) zur Abtrennung von Verunreinigungen und anschliessend eines Methylenchlorid: Methanol-Gemischs (9:1) zur Eluierung des gewünschten Produkts chromatographiert. Nach Entfernen des Lösungsmittels wird das erhaltene Produkt in Essigsäureäthylester gelöst und mit 30 ml 2-n. Salzsäure in das Hydrochlorid überführt, welches abgesaugt und mit Wasser und Essigsäureäthylester gewaschen wird. Nach dem Umkristallisieren aus 60 ml eines Aethanol-Wasser-Gemisches 1:1 erhält man 2,6-Dimethyl-(4-(3-nitrophenyl)-1,4-dihydropyridin-3-carbonsäuremethylester-5-N-[2-[4-[(p-fluorbenzoyl)-piperidino]-äthyl]-carboxamidhydrochlorid, welches sich ab 200° zersetzt.

Das Ausgangsmaterial kann wie folgt hergestellt werden:

Eine Lösung von 2,6-Dimethyl-4-(3-nitrophenyl-1,4-dihydropyridin-3,5-dicarbonsäure-monomethylester [Europäische Patentanmeldung 11.706], 3,4 g 1-Hydroxybenzotriazol und 4.6 g N,N'-Dicyclohexylcarbodiimid in 100 ml wasserfreiem Dimethylformamid wird während 16 Stunden bei 0-5° unter einer Stickstoffatmosphäre stehen gelassen. Der auskristallisierte N,N'-Dicyclohexylharnstoff wird abfiltriert. Man versetzt das gelbe Filtrat unter Eisbadkühlung mit 300 ml Wasser und rührt noch während 1 Stunde bei 0-5°, wobei das amorphe Rohprodukt abgeschieden wird. Es wird abfiltriert, der Filterrückstand mit 400 ml Wasser gewaschen und im Vakuum getrocknet. Zur weiteren Reinigung wird das Rohprodukt in 40 ml Essigsäureäthylester gelöst und während 1 Stunde bei 0-5° gerührt. Anschliessend entfernt man den auskristallisierten N,N'-Dicyclohexylharnstoff durch Filtration und dampft das Filtrat unter vermindertem Druck ein, wobei 2,6-Dimethyl-4-(3-nitrophenyl)-1,4-dihydropyridin-3,5-dicarbonsäure-3-(1-benzotriazolyl)-ester-5-methylester als hochviskoser Schaum erhalten wird.

Beispiel 2: Ein Gemisch von 11,9 g 2,6-Dimethyl-4-(3-nitrophenyl)-1,4-dihydropyridin-3,5-dicarbonsäure-methylester-2-chloräthylester, (DOS 2 407 115) 7,3 g 4-(p-Fluorbenzoyl)-piperidin-hydrochlorid, 11,4 ml N-Aethyl-N,N-diisopropylamin und 60 ml Xylol wird in einer Argonatmosphäre bei einer Badtemperatur von 150° während 8 Stunden gerührt. Anschliessend wird das Lösungsmittel am Rotationsverdampfer unter Vakuum entfernt, wonach man 30 g eines braunen Oels erhält, welches in 150 ml Essigsäureäthylester und 50 ml Wasser aufgenommen wird. Nach dem Abtrennen der organischen Phase wird diese mit Wasser und gesättigter wässriger Kochsalzlösung gewaschen und anschliessend über Natriumsulfat getrocknet. Nach dem Abdampfen zur Trockne verbleiben 20 g eines Oels, welches über 700 g Kieselgel Merck 9385 (0,04-0,063 mm) mit einem Hexan-Essigsäureäthylester-Gemisch 1:1 chromatographiert wird. Das eluierte Produkt wird in Aether gelöst und die Lösung tropfenweise mit 3-n. ätherischer

Chlorwasserstofflösung versetzt, wobei das 2,6-Dimethyl-4-(3-nitrophenyl)-1,4-dihydropyridin-3,5-dicarbonsäure-methylester-2-[4-(p-fluorenzoyl)-piperidino]-äthylester-hydrochlorid ausfällt, welches ab 136° unter Zersetzung schmilzt.

Beispiel 3: Ein Gemisch von 18,2 g 2,6-Dimethyl-4-(3-nitrophenyl)-1,4-dihydropyridin-3,5-dicarbonsäure-methylester-6-chlor-n-hexylester und 16,6 g 4-(p-Fluorbenzoyl)-piperidin wird in einem auf 140° aufgeheizten Bad mit einem Spatel bis zur Schmelze verrührt und 1 Stunde bei 140° erhitzt. Man kratzt die Reaktionsmasse aus dem Kolben und verrührt diese mit einem Gemisch von 250 ml Essigsäureäthylester, 350 ml Methylenchlorid und 100 ml 50%-iger wässriger Kaliumcarbonatlösung. Die organische Phase wird mit wässriger Kaliumcarbonatlösung gewaschen, über Kaliumcarbonat getrocknet, filtriert und eingedampft. Der Rückstand wird über 700 g Kieselgel chromatographiert, wobei als Fliessmittel: Hexan-Essigsäureäthylester-Methanol (4:4:0,5) verwendet wird. Man erhält so den 2,6-Dimethyl-4-(3-nitrophenyl)-1,4-dihydropyridin-3,5-dicarbonsäure-methylester-6-[4-(p-fluorbenzoyl)-piperidino]-n-hexylester, welcher mittels ätherischer Chlorwasserstofflösung in das amorphe Hydrochlorid überführt wird, welches ab 45° schmilzt.

Der als Ausgangsmaterial verwendete 2,6-Dimethyl-4-(3-nitrophenyl)-1,4-dihydropyridin-3,5-dicarbonsäure-methylester-6-chlor-n-hexylester kann analog der im Beispiel 11 der DOS 2 407 115 beschriebenen Verfahrensweise unter Verwendung von Acetessigsäure-6-chlor-n-hexylester hergestellt werden und wird als Rohprodukt weiterverarbeitet.

Beispiel 4: Ein Gemisch von 16,4 g 2,6-Dimethyl-4-(3-nitrophenyl)-1,4-dihydropyridin-3,5-dicarbonsäure-methylester-3-chlorpropylester und 16,6 g 4-(p-Fluorbenzoyl)-piperidin wird unter Argon während 1 Stunde in einem auf 145° erwärmten Bad gehalten. Nach dem Erhalten gibt man 100 ml Essigsäureäthylester und 50 ml 2N-Salzsäure hinzu und rührt bis zur völligen Lösung der Reaktionsmasse. Die zum Teil ölige wässrig-saure Phase wird mit 50%-iger Kaliumcarbonatlösung auf pH 10 eingestellt, mit Methylenchlorid versetzt und abgesaugt. Die Methylenchlorid-Phase des Filtrats wird abgetrennt, eingedampft und der Rückstand in Essigsäureäthylester gelöst, die Lösung über Natriumsulfat getrocknet und filtriert. Anschliessend filtriert man über 1 kg Kieselgel Merck 9385 unter Verwendung eines Hexan-Essigsäureäthylester-Methanol-Gemisches (4:2:0,5) als Fliessmittel. Der nach dem Aufarbeiten erhaltene 2,6-Dimethyl-4-(3-nitrophenyl)-1,4-dihydropyridin-3,5-dicarbonsäure-methylester-3-[4-(p-fluorbenzoyl)piperidino]-propylester wird mittels ätherischer Chlorwasserstofflösung in das Hydrochlorid überführt, welches nach dem Umkristallisieren aus Isopropanol bei 198-205° unter Gasentwicklung schmilzt.

Der als Ausgangsmaterial verwendete 2,6-Dimethyl-4-(3-nitrophenyl)-1,4-dihydropyridin-3,5-dicarbonsäure-methylester-3-chlorpropylester kann analog der im Beispiel 11 der DOS 2 407 115 beschriebenen Vefahrensweise unter Verwendung von Acetessigsäure-3-chloropropylester hergestellt werden und wird als Rohprodukt weiterverarbeitet.

Beispiel 5: Ein Gemisch von 11 g 2,6-Dimethyl-4-(3-nitrophenyl)-1,4-dihydropyridin-3,5-dicarbonsäure-methylester-2-chloräthylester, 6,4 g 4-(2-Thenoyl)-piperidin und 8,8 ml 1,8-Diazabicyclo[5,4,0]-undec-7-en wird während 1 Stunde in einem Bad von 150° unter Argon gerührt. Nach dem Erhalten löst man die Schmelze in 100 ml Essigsäureäthylester und wäscht die Lösung 3 mal mit 1N Salzsäure. Die wässrig-saure Lösung wird mit 50%-iger Kaliumcarbonatlösung auf pH 9-10 eingestellt und mit Essigsäureäthylester extrahiert. Die über Natriumsulfat getrocknete organische Phase wird eingedampft und der Rückstand in Aether gelöst. Durch Versetzen mit einer Lösung von Chlorwasserstoff in Aether erhält man das 2,6-Dimethyl-4-(3-nitrophenyl)-1,4-dihydropyridin-3,5-dicarbonsäure-methylester-2-[4-(2-thenoyl)-piperidino]-äthylester-hydrochlorid, welches bei 140-145° schmilzt; es sintert ab 120°.

Beispiel 6: Ein Gemisch von 8,4 g 2,6-Dimethyl-4-(2,3-dichlorphenyl)-1,4-dihydropyridin-3,5-dicarbonsäure-methylester-2-chloräthylester und 8,8 g 4-(p-Fluorbenzoyl)-piperidin wird während 1 Stunde bei einer Temperatur von 145° unter Argon gerührt. Nach dem Aufarbeiten wird der erhaltene Rückstand über 500 g Kieselgel chromatographiert, wonach man den 2,6-Dimethyl-4-(2,3-dichlorphenyl)-1,4-dihydropyridin-3,5-dicarbonsäure-methylester-2-[4-(p-flurobenzoyl)-piperidino]-äthylester in amorpher Form erhält, der im NMR-Spektrum folgende charakteristischen Banden zeigt: $^1$H-NMR($CDCl_3$): 2,3 und 2,4 [2S, 6H, 2 $H_3C$-C (2 bzw. 6)]; 3,6 [s, 3H, $H_3C$-OOC); 5,4 [s, 1H, H-c (4)], 7,1-8,1 (m, 7H, 7 arom. H). Rf-Wert: 0,40: (Hexan: Aethylacetat:Methanol = 4:2:1).

Der als Ausgangsstoff verwendete 2,6-Dimethyl-4-(2,3-dichlorphenyl)-1,4-dihydropyridin-3,5-dicarbonsäure-methylester-2-chloräthylester kann analog der im Beispiel 11 der DOS 2 407 115 beschriebenen Verfahrensweise unter Verwendung von 2,3-Dichlorbenzaldehyd erhalten werden und wird als Rohprodukt weiterverarbeitet.

Beispiel 7: Analog der im Beispiel 6 beschriebenen Vefahrensweise wird ein Gemisch von 4,4 g 4-(p-Fluorbenzoyl)-piperidin und 4,1 g 2,6-Dimethyl-4-(2,1,3-Benzoxadiazol-4-yl)-1,4-dihydropyridin-3,5-dicarbonsäure-methylester-3-chlorpropylester umgesetzt. Der nach Chromatographie über Kieselgel erhaltene Rückstand stellt den 2,6-Dimethyl-4-(2,1,3-benzoxadiazol-4-yl)-1,4-dihydropyridin-3,5-dicarbonsäuremethylester-3-[4-(p-fluorbenzoyl)-piperidino]-propylester dar, der im NMR-Spektrum folgende charakteristischen Banden zeigt: $^1$H-NMR ($CDCl_3$): 2,4 (2s, 6H, 2 $H_3C$-C (2 bzw. 6); 3,6 (s, 3H, $H_3C$-OOC); 4,1 (t, 2H, $H_3C$-OOC); 5,4 [s, 1H, H-C (4)]; 7,2-8,1 (m, 7H, 7 arom. H); Rf-Wert: 0,27 (Hexan: Aethylacetat: Methanol = 4:2:1).

Der als Ausgangsmaterial benötigte 2,6-Dimethyl-4-(2,1,3-Benzoxadiazol-4-yl)-1,4-dihydropyridin-3,5-dicarbonsäure-methylester-chlorpropylester kann analog der im Beispiel 11 der DOS 2 407 115

unter Verwendung von 3-Chlorpropylacetoacetat, 2,1,3-Benzoxadiazol-4-carboxaldehyd und Methyl-β-aminocrotonat erhalten werden, und wird als Rohprodukt weiterverarbeitet.

Beispiel 8: Tabletten enthaltend 20 mg an aktiver Substanz werden in folgender Zusammensetzung in üblicher Weise hergestellt:

Zusammensetzung:

| | |
|---|---|
| 2,6-Dimethyl-4-(3-nitrophenyl)-1,4-dihydropyridin-3-carbonsäuremethylester-5-N-[2-[4-p-fluorbenzoyl)-piperidino]-äthyl]-carboxamid | 20 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 145 mg |

Herstellung:

Der Wirkstoff wird mit einem Teil der Weizenstärke, mit Milchzucker und kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 145 mg Gewicht mit Bruchkerbe verpresst.

Beispiel 9: Tabletten enthaltend 1 mg an aktiver Substanz werden in folgender Zusammensetzung in üblicher Weise hergestellt:

Zusammensetzung:

| | |
|---|---|
| 2,6-Dimethyl-4-(3-nitrophenyl)-1,4-dihydropyridin-3,5-dicarbonsäure-methylester-2-[4-(p-fluorbenzoyl)-piperidino]-äthylester | 1 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 126 mg |

Herstellung:

Der Wirkstoff wird mit einem Teil der Weizenstärke, mit Milchzucker und kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 145 g Gewicht mit Bruchkerbe verpresst.

Beispiel 10: Kapseln enthaltend 10 mg an aktiver Substanz werden wie folgt auf übliche Weise hergestellt:

Zusammensetzung:

| | |
|---|---|
| 2,6-Dimethyl-4-(3-nitrophenyl)-1,4-dihydropyridin-3-carbonsäuremethylester-5-N-[2-[4-(p-fluorbenzoyl)-piperidino]-äthyl]-carboxamid | 2500 mg |
| Talkum | 200 mg |
| Kolloidale Kieselsäure | 50 mg |

Herstellung:
Die aktive Substanz wird mit Talkum und kolloidaler Kieselsäure innig gemischt, das Gemisch durch ein Sieb mit 0,5 mm Maschenweite getrieben und dieses in Portionen von jeweils 11 mg in Hartgelatinekapseln geeigneter Grösse abgefüllt.

Beispiel 11: Anstelle der in den Beispielen 8 bis 10 als aktive Substanz verwendeten Verbindungen können auch folgende Verbindungen der Formel I oder deren pharmazeutisch annehmbaren, nicht-toxischen Säureadditionssalze als Wirkstoffe in Tabletten, Dragées, Kapseln etc. verwendet werden: 2,6-Dimethyl-4-(3-nitrophenyl)-1,4-dihydropyridin-3,5-dicarbonsäure-methylester-6-[4-(p-fluorbenzoyl)-piperidino]-n-hexylester, 2,6-Dimethyl-4-(3-nitrophenyl)-1,4-dihydropyridin-3,5-dicarbonsäure-methylester-3-[4-(p-fluorbenzoyl)-piperidino]-propylester, 2,6-Dimethyl-4-(3-nitrophenyl)-1,4-dihydropyridin-3,5-dicarbonsäure-methylester-2-[4-(2-thenoyl)-piperidino]-äthylester, 2,6-Dimethyl-4-(2,3-dichlorphenyl)-1,4-dihydropyridin-3,5-dicarbonsäuremethylester-2-[4-(p-fluorbenzoyl)-piperidino]-äthylester, und 2,6-Dimethyl-4-(2,1,3-benzoxadiazol-4-yl)-1,4-dihydropyridin-3,5-dicarbonsäure-methylester-[4-(p-fluorbenzoyl)-piperidino]-propylester.

Patentansprüche

1. Verbindungen der Formel

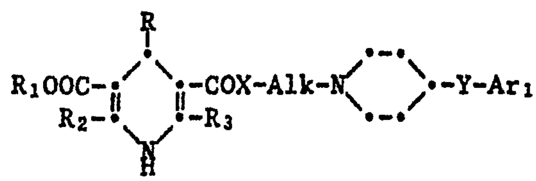

(I),

worin R einen carbocyclischen oder heterocyclischen Arylrest bedeutet, $R_1$ Niederalkyl darstellt, eine der Gruppen $R_2$ und $R_3$ für Niederalkyl und die andere für Niederalkyl, Cyan oder Amino steht, X Sauerstoff oder die Gruppe -NH- darstellt, und Alk Niederalkylen bedeutet, das die Gruppe X vom Ringstickstoffatom durch mindestens zwei Kohlenstoffatome trennt, Y für die Gruppen -CHOH- oder -(C=O)- steht und $Ar_1$ einen monocyclischen Aryl- oder Heteroarylrest darstellt, wobei, falls $R_1$, $R_2$ und $R_3$ jeweils Niederalkyl ist, X für Sauerstoff steht, Alk geradkettiges Alkylen mit 2 bis 4 Kohlenstoffatomen darstellt, Y die Gruppe -(C=O)- ist, und R die angegebene Bedeutung hat, $Ar_1$ verschieden von unsubstituiertem Phenyl ist, sowie Salze solcher Verbindungen.

2. Neue Verbindungen der Formel I, in welchen R für einen mono- oder bicyclischen, carbocyclischen Arylrest oder für einen fünf- oder sechsgliedrigen, ein bis und mit vier Ringstickstoffatome, ein Ringsauerstoff- oder Ringschwefelatom, oder ein oder zwei Ringstickstoffatome zusammen mit einem Ringsauerstoff- oder einem Ringschwefelatom als Ringglieder enthaltenden, monocyclischen Heteroarylrest steht, der über ein Ringkohlenstoffatom mit dem Kohlenstoffatom der 4-Stellung des 1,4-Dihydropyridinrings verbunden ist, und der gegebenenfalls einen ankondensierten Benzoring enthält, und insbesondere Phenyl, Naphthyl, Pyrryl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl, Triazinyl, Indolyl, Isoindolyl,

Benzimidazolyl, Benzoxadiazolyl, Benzofuranyl, Benzofurazanyl, Benzothienyl, Benzthiazolyl, 2,1,3-Benzthiadiazolyl, Chinolinyl oder Isochinolinyl bedeutet, wobei in diesen Resten Ringkohlenstoffatome gegebenenfalls durch Niederalkyl, Niederalkenyl, Niederalkinyl, Niederalkylen, Cycloalkyl, Phenyl und/oder Phenylniederalkyl, wobei Niederalkyl, Phenyl oder Phenylniederalkyl gegebenenfalls Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkylcarbamoyl und/oder Cyan, und cyclische Reste auch Niederalkyl, das seinerseits wie angegeben substituiert sein kann, als Substituenten enthalten können, und/oder durch Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Nitro, Amino, N-Niederalkyl-amino, N,N-Diniederalkylamino, N-Niederalkyl-N-phenylniederalkyl-amino, Niederalkylenamino, Oxaniederalkylenamino, Thianiederalkylenamino und/oder Azaniederalkylenamino, worin das Azastickstoffatom durch Niederalkyl, Phenyl oder Phenylniederalkyl substituiert sein kann, wobei diese Reste Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl und/oder Cyan, die cyclischen Reste auch Niederalkyl als Substituenten enthalten können, und/oder durch Niederalkanoylamino, Azido, Niederalkanoyl, Carboxy, Niederalkoxycarbonyl, Carbamoyl, N-Niederalkyl-carbamoyl, N,N-Diniederalkyl-carbamoyl, Cyan, Sulfo, Aminosulfonyl, N-Niederalkylaminosulfonyl, N,N-Diniederalkylaminosulfonyl, Niederalkylthio, Niederalkylsulfinyl und/oder Niederalkylsulfonyl, und/oder Ringstickstoffatome gegebenenfalls durch Niederalkyl, das als Substituenten gegebenenfalls Hydroxy, Niederalkoxy, Niederalkoxyniederalkoxy, Halogenniederalkoxy, Niederalkenyloxy, Halogenniederalkenylxy, Niederalkinyloxy, Niederalkylendioxy, Niederalkanoyloxy, Halogen, Carboxy, Niederalkoxycarbonyl, Carbamoyl, N-Niederalkylcarbamoyl, N,N-Diniederalkyl-carbamoyl oder Cyan enthalten kann, oder durch Hydroxy oder Oxido substituiert sein können, $R_1$ Niederalkyl ist, einer der Reste $R_2$ und $R_3$ für Niederalkyl und der andere für Niederalkyl, Cyan oder Amino steht, X Sauerstoff oder die Gruppe -NH- bedeutet, und Alk Niederalkylen darstellt, das die Gruppe X vom Ringstickstoffatom durch 2 bis 8 Kohlenstoffatome trennt, Y für die Gruppen der Formel -CHOH- oder insbesondere -(C=O)- steht, und $Ar_1$ einen monocyclischen carbocyclischen Aryl- oder Heteroarylrest darstellt, und insbesondere Phenyl, Naphthyl, Pyrryl, Furyl, Thienyl oder Pyridyl bedeutet, wobei in diesen Resten Ringkohlenstoffatome gegebenenfalls durch Niederalkyl, Niederalkenyl, Niederalkinyl, Niederalkylen, Cycloalkyl, Phenyl und/oder Phenylniederalkyl substituiert sind, wobei Niederalkyl, Phenyl oder Phenylniederalkyl als Substituenten Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl und/oder Cyan, und cyclische Reste auch Niederalkyl, das seinerseits wie angegeben substituiert sein kann, enthalten können, und/oder durch Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Nitro, Amino, N-Niederalkyl-amino, N,N-Diniederalkylamino, N-Niederalkyl-N-phenylniederalkyl-amino, Niederalkylenamino, Oxoniederalkylenmino, Thianiederalkylenamino und/oder Azaniederalkylenamino, worin das Azastickstoffatom durch Niederalkyl, Phenyl oder Phenylniederalkyl substituiert sein kann, wobei diese Reste Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkylcarbamoyl und/oder Cyan, die cyclischen Reste auch Niederalkyl als Substituenten enthalten können, und/oder durch Niederalkanoylamino, Azido, Niederalkanoyl, Carboxy, Niederalkoxycarbonyl, Carbamoyl, N-Niederalkyl-carbamoyl, N,N-Diniederalkyl-carbamoyl, Cyan, Sulfo, Aminosulfonyl, N-Niederalkylaminosulfonyl, N,N-Diniederalkylamino-

sulfonyl, Niederalkylthio, Niederalkylsulfinyl und/oder Niederalkylsulfonyl, und/oder Ringstickstoffatome gegebenenfalls durch Niederalkyl, das als Substituenten gegebenenfalls Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkylcarbamoyl, N,N-Diniederalkyl-carbamoyl oder Cyan als Substituenten enthalten kann, oder durch Hydroxy oder Oxido substituiert sein können, wobei falls $R_1$, $R_2$ und $R_3$ jeweils Niederalkyl ist, X für Sauerstoff steht, Alk geradkettiges Alkylen mit 2 bis 4 Kohlenstoffatomen darstellt, Y die Gruppe -(C=O)- ist, und R die angegebene Bedeutung hat, $Ar_1$ verschieden von unsubstituiertem Phenyl ist, sowie Salze solcher Verbindungen.

3. Verbindungen der Formel I, worin R und $Ar_1$ jeweils für Phenyl oder Naphthyl stehen, das gegebenenfalls durch Niederalkyl, Phenyl und/oder Phenylniederalkyl, wobei solche Reste ihrerseits Hydroxy, Niederalkoxy, Halogenniederalkoxy, Niederalkylendioxy, Halogen, Carboxy, Niederalkoxycarbonyl und/oder Cyan, die cyclischen Reste auch Niederalkyl als Substituenten enthalten können, und/oder durch Hydroxy, Niederalkoxy, Halogenniederalkoxy, Niederalkenyloxy, Halogenniederalkenyloxy, Niederalkylendioxy, Halogen, Nitro, Amino, N-Niederalkylamino, N,N-Diniederalkylamino, Niederalkanoylamino, Carboxy, Niederalkoxycarbonyl, Carbamoyl, Cyan, Sulfo, Aminosulfonyl, N-Niederalkylaminosulfonyl, N,N-Diniederalkylaminosulfonyl, Niederalkylthio, Niederalkylsulfinyl und/oder Niederalkylsulfonyl substituiert ist, R zudem jeweils für über ein Ringkohlenstoffatom gebundenes Pyrryl, Furyl, Thienyl, Pyridyl, 1-Oxido-pyridyl, Imidazolyl, Benzofurazanyl oder Benzoxadiazolyl und $Ar_1$ zudem für über ein Ringkohlenstoffatom gebundenes Pyrryl, Furyl, Thienyl oder Pyridyl stehen kann, wobei solche Reste R und/oder $Ar_1$ gegebenenfalls, wie für einen Phenyl- oder Naphthylrest R bzw. $Ar_1$ angegeben, substituiert sind, und insbesondere Niederalkyl, Niederalkoxy, Halogen und/oder gegebenenfalls durch Niederalkyl, Niederalkoxy, Halogen und/oder Nitro substituiertes Phenyl als Substituenten enthalten können, $R_1$ Niederalkyl ist, $R_2$ und $R_3$ jeweils Niederalkyl

bedeuten, oder eine der Gruppen $R_2$ und $R_3$ Niederalkyl ist und die andere Niederalkyl oder Cyano darstellt, X Sauerstoff oder die Gruppe -NH- bedeutet, und Alk für die Gruppe $-(CH_2)_n-$ steht, worin n Werte von 2 bis 6 darstellt, und Y für die Gruppen der Formel -CHOH- oder in erster Linie -(C=O)- steht, wobei, falls $R_1$, $R_2$ und $R_3$ jeweils Niederalkyl ist, X für Sauerstoff steht, n Werte von 2 bis 4 darstellt, Y die Gruppe -(C=O)- ist, und R die angegebene Bedeutung hat, $Ar_1$ verschieden von unsubstituiertem Phenyl ist, sowie Salze, solcher Verbindungen .

4. Verbindungen der Formel I, worin R und $Ar_1$ jeweils für Phenyl steht, das gegebenenfalls durch Niederalkyl, z.B. Methyl, Hydroxy, Niederalkoxy, z.B. Methoxy oder Aethoxy, Halogenniederalkoxy, z.B. Difluormethoxy oder 2-Chlor-1,1,2-trifluoräthoxy, Halogenniederalkenyloxy, z.B. 1,2-Dichlor-vinyloxy, Niederalkylendioxy, z.B. Methylendioxy, Halogen, z.B. Fluor, Chlor oder Brom, Trifluormethyl, Nitro, Niederalkanoylamino, z.B. Acetylamino, gegebenenfalls durch gegebenenfalls verestertes oder veräthertes Hydroxy wie Halogen, z.B. Fluor oder Chlor, oder Niederalkoxy, z.B. Methoxy substituiertes Phenyl oder Phenylniederalkyl, und/oder Cyan substituiert ist, wobei ein Phenylrest R bzw. $Ar_1$ einen oder mehrere, gleiche oder verschiedene Substituenten aufweisen kann, R zudem Pyridyl, z.B. 2-, 3- oder 4-Pyridyl, Furyl, z.B. 2-Furyl, 1-Oxido-pyridyl, z.B. 1-Oxido-3-pyridyl, Thienyl, z.B. 2-Thienyl, R auch Benzofurazanyl, z.B. 4-Benzofurazanyl, und $Ar_1$ zudem Pyridyl, z.B. 2-, 3- oder 4-Pyridyl, Furyl, z.B. 2-Furyl oder Thienyl, z.B. 2-Thienyl bedeuten kann, wobei solche Reste R bzw. $Ar_1$ durch Niederalkyl, z.B. Methyl, oder Halogen, z.B. Fluor oder Chlor, substituiert sein können, $R_1$, $R_2$ und $R_3$ jeweils Niederalkyl bedeuten, X insbesondere für Sauerstoff, ferner für die Gruppe -NH- steht, Alk die Gruppe $-(CH_2)_n-$ darstellt, worin n Werte von 2 bis 4 bedeutet, und Y für die Gruppen der Formel -CHOH- oder insbesondere -(C=O)- steht, wobei falls $R_1$, $R_2$ und $R_3$ jeweils Niederalkyl ist, X für Sauerstoff steht, n Werte von 2 bis 4 darstellt, Y die Gruppe -(C=O)- ist, und R die angegebene Bedeutung hat, $Ar_1$ verschieden von unsubstituiertem Phenyl ist, sowie Salze solcher Verbindungen.

5. Verbindungen der Formel I, worin R für unsubstituiertes oder vorzugsweise durch Niederalkyl, z.B. Methyl, Niederalkoxy, z.B. Methoxy, Halogenniederalkoxy oder Halogenniederalkenyloxy, worin Halogen eine Atomnummer bis und mit 35 hat und insbesondere Fluor oder Chlor ist, z.B. Difluormethoxy, Halogen mit einer Atomnummer bis und mit 35, Trifluormethyl, Nitro und/oder Cyan mono-oder di-substituiertes Phenyl steht, wobei Substituenten üblicherweise die 2- und/oder 3-Stellung einnehmen, $R_1$, $R_2$ und $R_3$ jeweils Niederalkyl bedeuten, X insbesondere für Sauerstoff, ferner für die Gruppe -NH- steht, Alk die Gruppe $-(CH_2)_n-$ darstellt, worin n Werte von 2 bis 4 bedeutet, Y für die Gruppen der Formel -CHOH- oder insbesondere -(C=O)- steht, und $Ar_1$ gegebenenfalls durch Halogen, mit einer Atomnummer bis und mit 35 substituiertes Phenyl oder Pyridyl, z.B. 2-Pyridyl, 3-Pyridyl oder 4-Pyridyl bedeutet, wobei, falls $R_1$, $R_2$ und $R_3$ jeweils Niederalkyl ist, X für Sauerstoff steht, n Werte von 2 bis 4 darstellt, Y die Gruppe -(C=O)- ist, und R die angegebene Bedeutung hat, $Ar_1$ verschieden von unsubstituiertem Phenyl ist, sowie Salze, insbesondere pharmazeutisch verwendbare, nicht-toxische Säureadditionssalze solcher Verbindungen.

6. Verbindungen der Formel I, worin R für durch Halogen mit einer Atomnummer bis und mit 35, z.B. Fluor, Chlor oder Brom, mono- oder disubstituiertes oder durch Trifluormethyl, Nitro oder Cyan monosubstituiertes Phenyl steht, wobei Substituenten die 2- und/oder 3-Stellung einnehmen, $R_1$ Niederalkyl, insbesondere Methyl oder Aethyl, und $R_2$ und $R_3$ jeweils Niederalkyl, in erster Linie Methyl, bedeuten, X für Sauerstoff steht, Alk die Gruppe $-(CH_2)_n-$ darstellt, worin n Werte von 2 bis 3 bedeutet, Y für die Gruppe der Formel -(C=O)- steht, und $Ar_1$ gegebenenfalls durch Halogen mit einer Atomnummer bis und mit 35, z.B. Fluor, substituiertes Phenyl ist, wobei, falls $R_1$, $R_2$ und $R_3$ jeweils Niederalkyl ist, X für Sauerstoff steht, n die angegebene Bedeutung hat und R die angegebene Bedeutung hat, $Ar_1$ verschieden von unsubstituiertem Phenyl ist, sowie Salze, insbesondere pharmazeutisch verwendbare, nicht-toxische Säureadditionssalze solcher Verbindungen.

7. Verbindungen der Formel 1, worin R für durch Halogen mit einer Atomnummer bis und mit 35, z.B. Fluor, Chlor oder Brom, mono- oder disubstituiertes, oder vorzugsweise durch Nitro, ferner durch Cyan monosubstituiertes Phenyl steht, wobei Substituenten die 2- und/oder 3-Stellung einnehmen, $R_1$ Niederalkyl, insbesondere Methyl oder Aethyl, und $R_2$ und $R_3$ jeweils Niederalkyl in erster Linie Methyl bedeuten, X für Sauerstoff steht, Alk die Gruppe $-(CH_2)_n-$ darstellt, worin n die Werte von 2 bis 3 bedeutet, Y für die Gruppe -(C=O)- steht, und $Ar_1$ durch Halogen mit einer Atomnummer bis und mit 35, besonders durch Fluor, in erster Linie in 4-Stellung substituiertes Phenyl ist, sowie Salze, insbesondere pharmazeutisch verwendbare, nicht-toxische Säureadditionssalze solcher Verbindungen.

8. 2,6-Dimethyl-(4-(3-nitrophenyl)-1,4-dihydropyridin-3-carbonsäuremethylester-5-N-[2-[4-[(p-fluorbenzoyl)-piperidino]-äthyl]-carboxamid und dessen Salze.

9. 2,6-Dimethyl-4-(3-nitrophenyl)-1,4-dihydropyridin-3,5-dicarbonsäure-methylester-2-[4-(p-fluorbenzoyl)-piperidino]-äthylester und dessen Salze.

10. 2,6-Dimethyl-4-(3-nitrophenyl)-1,4-dihydropyridin-3,5-dicarbonsäure-methylester-6-[4-(p-fluorbenzoyl)-piperidino]-n-hexylester und dessen Salze.

11. 2,6-Dimethyl-4-(3-nitrophenyl)-1,4-dihydropyridin-3,5-dicarbonsäure-methylester-3-[4-(p-fluorbenzoyl)-piperidino]-propylester und dessen Salze.

12. 2,6-Dimethyl-4-(3-nitrophenyl)-1,4-dihydropyridin-3,5-dicarbonsäure-methylester-2-[4-(2-thenoyl)-piperidino]-äthylester und dessen Salze.

13. 2,6-Dimethyl-4-(2,3-dichlorphenyl)-1,4-dihydropyridin-3,5-dicarbonsäure-methylester-2-[4-(p-fluorbenzoyl)-piperidino]-äthylester und dessen Salze.

14. 2,6-Dimethyl-4-(2,1,3-benzoxadiazol-4-yl)-1,4-dihydropyridin-3,5-dicarbonsäure-methylester-3-[4-(p-fluorbenzoyl)-piperidino]-propylester und dessen Salze.

15. Pharmazeutisch verwendbare nicht-toxische Säureadditionssalze von Verbindungen der Ansprüche 1-14.

16. Verbindungen gemäss den Ansprüchen 1-15 mit coronardilatatorischen und antihypertensiven Eigenschaften.

17. Verbindungen gemäss Ansprüchen 1-15 zur Anwendung in einem Verfahren zur therapeutischen Behandlung des menschlichen oder tierischen Körpers.

18. Pharmazeutische Präparate enthaltend Verbindungen der Ansprüche 1-15.

19. Verwendung von Verbindungen der gemäss den Ansprüchen 1-15 zur Herstellung von pharmazeutischen Präparaten.

20. Verwendung von Verbindungen gemäss den Ansprüchen 1-15 zur Herstellung von Arzneimitteln als Coronardilitatoren und Antihypertensiva zur Behandlung von cardiosvaculären Krankheitszuständen, wie Angina pectoris und ihre Folgezustände, Gefässspasmen, zentrale und periphere Durchblutungsstörungen, hohem Blutdruck, Arrythmien und Herzinsuffizienz, ferner zur Anwendung als Hemmer der Plättchenaggregation.

21. Verfahren zur Herstellung von Verbindungen der Formel I gemäss Anspruch 1 und Salzen davon, dadurch gekennzeichnet, das man

a) eine Verbindung der Formel

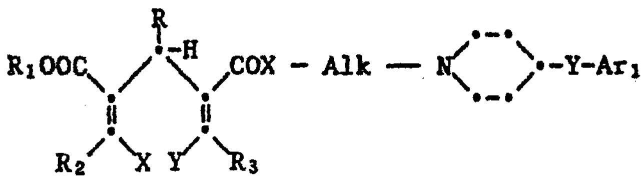

(II),

worin einer der Reste X und Y für die Gruppe der Formel $-NH_2$ steht und der andere Hydroxy oder die Gruppe der Formel $-NH_2$ bedeutet, oder ein Tautomeres davon oder ein entsprechendes Tautomerengemisch ringschliesst, oder

b) eine Verbindung der Formel R-CHO (III) oder ein reaktionsfähiges funktionelles Derivat davon mit einer Verbindung der Formel

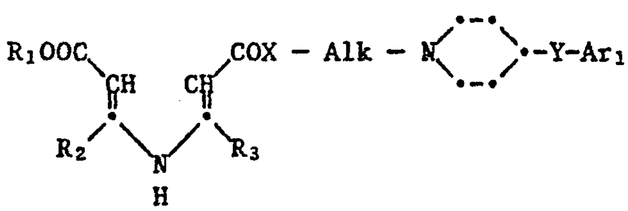

(IV)

oder einem Tautomeren davon oder einem entsprechenden Tautomerengemisch umsetzt, oder

c) in einer Verbindung der Formel

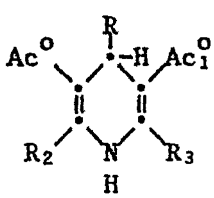

(V),

in welcher einer der Reste $Ac^{o}$ und $Ac_{1}^{o}$ eine in die Gruppe $-COOR_1$ oder in den Rest der Formel

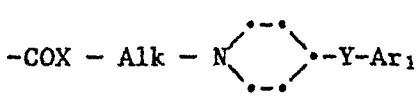

(Va)

überführbare Gruppe bedeutet, und der andere die Gruppe $-COOR_1$ oder die Gruppe der Formel Va bedeutet, den Rest $Ac^o$ bzw. $Ac_1^o$ in die Gruppe $-COOR_1$ bzw. in einen Rest der Formel Va überführt, oder

d) eine Verbindung der Formel

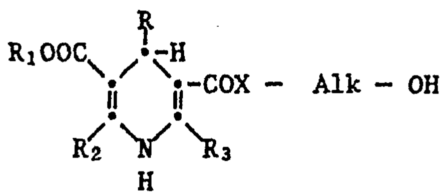

(VI)

oder einen reaktionsfähigen Ester davon, mit einer Verbindung der Formel

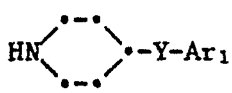

(VIa)

umsetzt, oder

e) eine Verbindung der Formel

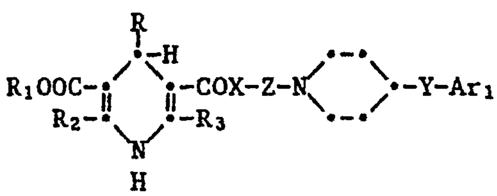

(VII)

worin Z eine mittels Reduktion in die Gruppe Alk überführbare Gruppe darstellt, die Gruppe Z zur Gruppe Alk reduziert, wobei die Ausgangsstoffe der Formel II bis VII sofern sie reaktionsfähige Derivate bilden können oder salzbildende Eigenschaften aufweisen, auch in Form von reaktionsfähigen Derivaten oder in Form ihrer Salze verwendet werden können, und die Gruppen R, $R_1$, $R_2$, $R_3$, X, Y, $Ar_1$ und Alk die unter der Formel I angegebenen Bedeutungen haben, und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz

umwandelt, und/oder, wenn erwünscht, eine erhaltene freie Verbindung der Formel I in ein Salz umwandelt, und/oder, wenn erwünscht, ein erhaltenes Isomerengemisch in die individuellen Isomeren auftrennt.

22. Die nach dem Verfahren des Anspruches 24 erhältlichen Verbindungen.

FO 7.4 EJA (HM)/hc*eg*